# EUROPEAN PATENT SPECIFICATION

(11) **EP 1 595 918 B1**
(45) Date of publication and mention of the grant of the patent: **06.08.2008**
(21) Application number: 05252515.1
(22) Date of filing: 22.04.2005
(51) Int. Cl.: C08L 59/00, C08K 5/24, C08K 5/25, C08L 79/00, A61L 9/01

(54) **Deodorizing agent and resin composition**
Desodorierungsmittel und Harzzusammensetzung
Agent désodorisant et composition de résine

(30) Priority: 30.04.2004 JP 2004136536
(43) Date of publication of application: 16.11.2005
(73) Proprietor: Polyplastics Co., Ltd., Tokyo 108-8280 (JP)
(72) Inventor: Harashina, Hatsuhiko, Fuji-shi Shizuoka 416-8533 (JP)
(74) Representative: Peel, James Peter

(56) References cited:
- EP-A- 1 522 554
- US-A- 3 574 786
- US-A- 5 096 951
- US-A- 6 147 146

## Description

### TECHNICAL FIELD

The present invention relates to a deodorizing agent useful for deodorizing an aldehyde odor (particularly a formaldehyde odor), and a resin composition containing the deodorizing agent.

### TECHNICAL FIELD

Aldehydes, in particular formaldehyde, have been used as a raw material of a resin (for example, a urea resin, a melamine resin, a phenolic resin, and a polyacetal resin) in various fields such as a building material for residential use, an adhesive, a coating agent and a shaped article. In the case of using such a resin, however, it happens that adverse effects on living environment and occupier's health due to an irritating odor caused by emission of formaldehyde from the resin become problems.

As a deodorizing agent for an aldehyde, for example, an activated carbon, a melamine compound, a urea compound, an amino acid, a polyhydric alcohol, and others have been known. Moreover, it has been known that some of carboxylic acid hydrazides have an odor eliminating property to aldehydes. For example, Japanese Patent Application Laid-Open No. 36681/1998 (JP-10-36681A) discloses that a resin composition containing a synthetic resin and a hydrazide compound (e.g., a monohydrazide compound including salicylic acid hydrazide, p-hydroxybenzoic acid hydrazide and naphthoic acid hydrazide; and a polyhydrazide compound including a polyacrylic acid hydrazide) has an odor eliminating effect to acetaldehyde or formaldehyde. Further, it has been also known that aldehydes can be removed or eliminated by using a composition comprising, as an active ingredient, a combination of such a hydrazide compound and a metal salt of a weak acid (Japanese Patent Application Laid-Open No. 152979/2000 (JP-2000-152979A), WO01/62309 description), a combination of the hydrazide compound and a metal compound (e.g., a halide, a nitrate salt, and an oxide) (WO01/39808 description) or a combination of the hydrazide compound and an ammonium compound (WO01/62309 description).

These conventional aldehyde-deodorizing agents, however, have an insufficient performance for inhibiting the aldehyde emission or heat resistance. It is therefore impossible to trap aldehyde absolutely to reduce smells, or to maintain a trapping ability or an odor-eliminating ability over a long period of time due to a high volatility and/or exhalation property or exudation (blooming) property.

Moreover, among resins emitting (or generating) aldehydes, a thermoplastic resin, such as a polyacetal resin or a polyvinyl acetal resin, is often molded (or pelletized) by hot melting, and further, in the light of productivity, such a obtained shaped article (including a pellet) is put (or wrapped) into a package in a high temperature state. In such a case, aldehyde is easily generated as the temperature of the shaped article is raised.

Incidentally, U.S. Patent No. 3,660,438 specification discloses that an alkyl-hydroxy-phenylalkanoyl hydrazine (e.g., N,N'-bis-β-(3,5-di-t-butyl-4-hydroxyphenyl)-propionyl-hydrazine) is useful as a stabilizer for a resin undergone an oxidative decomposition (including a polypropylene, a high impact polystyrene, a polyacetal, and others). U.S. Patent No. 4,007,183 specification discloses 2,4-bis or 2,4,6-tris(salicylic acid hydrazino)-1,3,5-triazine as a stabilizer for a polyolefin. Japanese Patent Application Laid-Open No. 6535/1980 (JP-55-6535A) discloses to improve the quality of paper in paper manufacturing, by allowing, in a pipe slurry, (a) a water-soluble polymer having an acid hydrazide group, or an adduct of the polymer with a water-soluble inorganic salt of an alkaline earth metal, and (b) a heavy metal ion capable of chelating with the water-soluble polymer or an adduct thereof, to coexist. U.S. Patent No. 2,615,862 specification discloses to produce a filament-formable polyhydrazide by allowing a dicarboxylic acid or a derivative thereof to react with a dihydrazide in an organic solvent at a polymerization temperature. U.S. Patent No. 2,395,642 specification discloses that a polymer obtained from a dihydrazide of a dicarboxylic acid is useful in manufacture of a filament, a fiber, a film, and others, and is also useful as a reagent for enhancing dye reception of a synthetic fiber. U.S. Patent No. 3,130,182 specification discloses that a high-molecular weight aromatic condensed polymer, being obtained from hydrazine and having a fiber- or film-forming property, has a high melting temperature, is excellent in solubility and possesses usual stability.

It is therefore an object of the present invention to provide a deodorizing agent (or a deodorizing agent composition) and a resin composition, which can efficiently inhibit an aldehyde odor and is excellent in a heat resistance (a stability for heat fading), a process for producing the resin composition, and a shaped article formed from the resin composition.

It is another object of the present invention to provide a deodorizing agent (or a deodorizing agent composition) and a resin composition, which can certainly trap (or capture) an aldehyde without deteriorating resin properties in the case using the agent or composition obtained by mixing with a resin, a process for producing the resin composition, and a shaped article formed from the resin composition.

It is still another object of the present invention to provide a deodorizing agent (or a deodorizing agent composition) and a resin composition, which ensures to inhibit bleed out or volatilization and exhalation and to trap an aldehyde or eliminate an aldehyde odor over a long period of time, a process for producing the resin composition, and a shaped article formed from the resin composition.

It is a further object of the present invention to provide a deodorizing agent (or a deodorizing agent composition) which can drastically inhibit an aldehyde emission from an aldehyde-generating resin in the case of being mixed with such a resin.

### DISCLOSURE OF THE INVENTION

The inventor of the present invention made intensive studies to achieve the above objects and finally found that a specific hydrazine compound has an aldehyde odor-eliminating property, and in addition a heat resistance (a stability for heat fading), a non-blooming property and a low volatility, and can maintain a high aldehyde odor-eliminating property even in the case of using the compound in combination with a resin. The present invention was accomplished based on the above findings.

According to the present invention there is provided an aldehyde odor-deodorizing agent which comprises at least one hydrazine compound selected from the group consisting of a polycarboxylic acid hydrazide having an aryl group, a hydrazide of an arylalkanecarboxylic acid (A1), a non-acrylic polyhydrazide (A2), and an aminourazole compound (A3),
wherein the polycarboxylic acid hydrazide having an aryl group and the hydrazide of the arylalkanecarboxylic acid (A1) have at least one hydroxyl group or a group derived from the hydroxy group on the aryl group(s),
the polycarboxylic acid hydrazide having an aryl group and the hydrazide of the arylalkanecarboxylic acid (A1) have at least one free hydrazino group, and
the non-acrylic polyhydrazide (A2) comprises a polyhydrazide shown by the following formula (2): 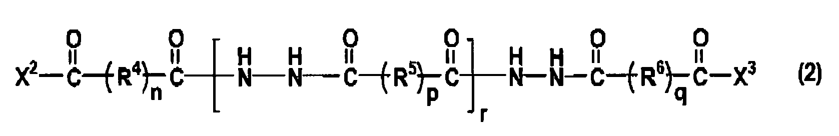 wherein R⁴ to R⁶ are the same or different and each represents a divalent hydrocarbon group or heterocyclic group, "n", "p" and "q" are the same or different and each denotes 0 or 1, "r" denotes an integer of not smaller than 0;
x² and X³ are the same or different and each represents a halogen atom, an amino group, a hydrazino group, or a group -OX⁴ wherein X⁴ represents a hydrogen atom, a hydrocarbon group, an alkali metal atom, or an alkaline earth metal atom, and with the proviso that at least one of X² and X³ is a hydrazino group.

Such a deodorizing agent efficiently reduces an aldehyde odor.

The polycarboxylic acid hydrazide having an aryl group and the hydrazide of the arylalkanecarboxylic acid (A1) have at least one hydroxyl group or a derivative thereof (e.g., an alkoxy group and an acyloxy group) on the aryl group. The polycarboxylic acid hydrazide having an aryl group and the hydrazide of the arylalkanecarboxylic acid (A1) may have at least one free hydrazino group (-NHNH₂). The polycarboxylic acid hydrazide having an aryl group and the hydrazide of the arylalkanecarboxylic acid (A1) may comprise a carboxylic acid hydrazide shown by the following formula (1): 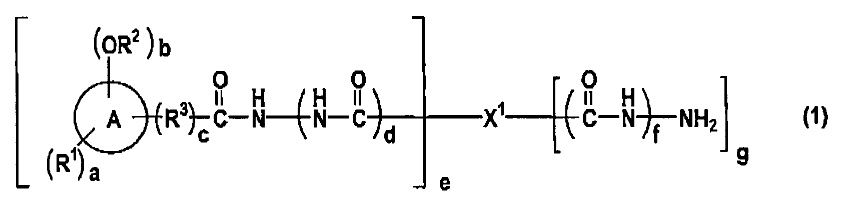 wherein R¹ represents a hydrocarbon group, R² represents a hydrogen atom, an alkyl group or an acyl group, R³ represents an alkylene group, a ring A represents an aromatic ring, X¹ represents a direct bond or a polyvalent organic group; "a" denotes an integer of 0 to 6, "b" denotes an integer of 1 to 4; "c" denotes 0 or 1, "d" is 1 when "c" is 0, "d" is 0 or 1 when "c" is 1; "e" denotes an integer of 1 to 5, "f" denotes 0 or 1 and "g" is 1 or 2 (preferably 1). In the formula (1), R¹ may be an alkyl group, a cycloalkyl group, a cycloalkylalkyl group, an aryl group, or an aralkyl group, R² may be a hydrogen atom, R³ may be a direct bond or a C₁₋₆alkylene group, "a" may be an integer of 0 to 4, "b" may be an integer of 1 to 3, and X¹ may be a direct bond, a di- to pentavalent hydrocarbon group, or a di- or trivalent (or bi-or tervalent) heterocyclic group. Incidentally, in the formula (1), the total number of "e" and "g" may be an integer of 2 to 5. The polycarboxylic acid hydrazide (A1) may comprise a compound shown in the following formula (1a): 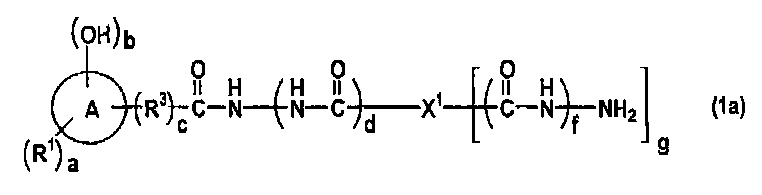 wherein R¹, R³, A, X¹, "a" to "d" , "f" and "g" have the same meanings as defined above.

The non-acrylic polyhydrazide (A2) comprises a polyhydrazide shown by the following formula (2): 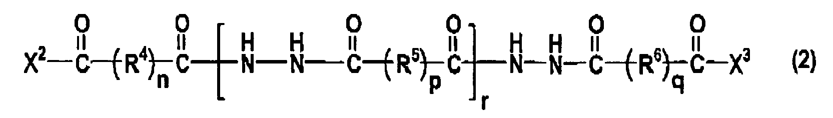 wherein R⁴ to R⁶ are the same or different and each represents a divalent hydrocarbon group or heterocyclic group, "n", "p" and "q" are the same or different and each denotes 0 or 1, "r" denotes an integer of not smaller than 0; X² and X³ are the same or different and each represents a halogen atom, an amino group, a hydrazino group (-NHNH₂), or a group -OX⁴ (in the formula, X⁴ represents a hydrogen atom, a hydrocarbon group, an alkali metal atom, or an alkaline earth metal atom), with the proviso that at least any one of the X² and X³ is a hydrazino group. In the formula (2), R⁴, R⁵ and R⁶ may be alkylene groups. The polyhydrazide shown by the formula (2) may have a number average molecular weight of 300 to 10,000.

The aminourazole compound (A3) may comprise a compound shown by the following formula (3): 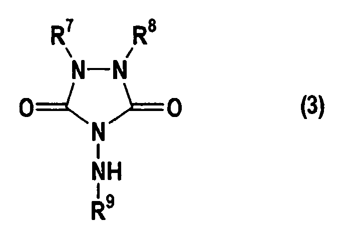 wherein R⁷, R⁸ and R⁹ are the same or different and each represents a hydrogen atom, an alkyl group, a cycloalkyl group, an aryl group or an aralkyl group; or a salt thereof. The aminourazole compound may comprise 4-aminourazole in which R⁷, R⁸ and R⁹ in the formula (3) are hydrogen atoms, a metal salt thereof, or a salt thereof with a nitrogen-containing compound.

The deodorizing agent may further comprise at least one deodorizing auxiliary selected from the group consisting of a basic nitrogen-containing compound, a weak acid or a metal salt thereof, a metal compound (e.g., a (hydrous) metal compound, and a metal chloride), an alcohol, a polyphenol and an adsorbent inorganic compound. Such a deodorizing agent is sometimes referred to as a deodorizing agent composition. The proportion of the deodorizing auxiliarymaybe about 0.01 to 10,000 parts by weight relative to 100 parts by weight of the deodorizing agent.

The present invention also includes a resin composition (including an odor-eliminating resin composition) comprising at least the deodorizing agent and a resin. The resin may comprise at least one member selected from the group consisting of a polyacetal resin, an olefinic resin, a halogen-containing vinyl-series resin, a styrenic resin, an acrylic resin, a polyester-series resin, and a polyurethane-series resin. The resin composition may contain about 0.01 to 30 parts by weight of the deodorizing agent relative to 100 parts by weight of the resin.

The present invention also includes a polyacetal resin composition comprising a polyacetal resin and the hydrazine compound. In the polyacetal resin composition, a pellet of the polyacetal resin may coexist with a master batch containing the hydrazine compound.

The resin composition (e.g., the odor-eliminating resin composition, and the polyacetal resin composition) may further comprise at least one member selected from the group consisting of an antioxidant, a heat stabilizer (a or a heat resistance stabilizer), a processing stabilizer, a weather (light)-resistant stabilizer, an impact resistance improver, a gloss-controlling agent, a slip-improving agent, a coloring agent, and a filler.

Moreover, the present invention includes a process for producing a polyacetal resin composition, which comprises melt-mixing a polyacetal resin and the hydrazine compound with an extruder, wherein at least the hydrazine compound is fed from a side feed port of the extruder and mixed with the polyacetal resin. Further, the present invention includes a process for producing a polyacetal resin composition, which comprises melt-mixing a polyacetal resin and the hydrazide compound with an extruder, wherein the average retention time in the extruder is not longer than 300 seconds (e.g., about 5 to 300 seconds).

The shaped article of the polyacetal resin of the present invention is formed from the polyacetal resin composition. In the shaped article, (1) the emission of formaldehyde from the shaped article which is maintained in a closed space for 24 hours at a temperature of 80°C may be not more than 1.0 µg (e.g., about 0 to 1.0 µg) per one cm² of the surface area of the article, and/or (2) the emission of formaldehyde from the shaped article which is maintained in a closed space for 3 hours at a temperature of 60°C under saturated humidity may be not more than 1. 2 µg (e.g., about 0 to 1.2 µg) per one cm² of the surface area of the article. The shaped article may be an automotive part, an electric and/or electronic device part, an architectural and/or pipeline part, a household utensil part, a medical device part, an office automation device part, a food grade part, or a photographic part.

### DETAILED DESCRIPTION OF THE INVENTION

The deodorizing agent of the present invention comprises a hydrazine compound excluding a hydroxybenzoic acid hydrazide, a naphthoic acid hydrazide and a polyacrylic acid hydrazide. The deodorizing agent can efficiently trap (or catch) an aldehyde to effectively eliminate an aldehyde odor. A hydroxyl group or a group derived from the hydroxyl group is hereinafter, sometimes referred to as a derivative group [for example, an alkoxy group such as methoxy, ethoxy, isopropoxy or t-butoxy (e.g., a linear chain or branched chain C₁₋₆alkoxy group); and an acyloxy group (e.g., an aliphatic C₂₋₆acyloxy group such as acetoxy or ethylcarbonyloxy; and an aromatic C₆₋₁₀aryl- carbonyloxy group such as benzoyloxy group)] (throughout this description, an aryl group (or moiety) having a hydroxyl group or a derivative group thereof may be simply referred to as a hydroxyaryl group (or a hydroxyaryl)).

Examples of the polycarboxylic acid hydrazide may include a carboxylic acid hydrazide having a hydroxyaryl group-containing carboxylic acid unit, a hydrazine unit and a polycarboxylic acid unit. Moreover, the hydrazide of a hydroxyarylalkanecarboxylic acid may embrace a carboxylic acid hydrazide having a hydroxyarylalkane carboxylic acid unit and a hydrazine unit. Further, the polycarboxylic acid hydrazide having a hydroxyaryl group and the hydrazide of a hydroxyarylalkanecarboxylic acid (A1) preferably has at least one free hydrazino group (-NHNH₂).

A preferred compound shown in the formula (1) is a compound in which the group -OR² is a hydroxyl group (that is, R² is a hydrogen atom). For example, such a compound may be represented by the following formula (1a): 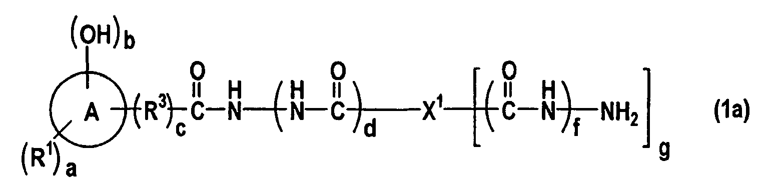 wherein R¹, R³, A, X¹, "a" to "d", "f" and "g" have the same meanings as defined above.

In the formulae (1) and (1a), the aromatic ring represented by the ring A may include a C₆₋₁₄aromatic ring such as a benzene ring or a naphthalene ring, and in addition, a bisaryl compound [for example, a bisaryl compound having a carbon number of about 12 to 24, e.g., biphenyl and a bisarylalkane (e.g., a bisC₆₋₁₀aryl-C₁₋₄alkane such as diphenylmethane or 2,2-diphenylpropane)]. As the aromatic ring A, a C₆₋₁₀aromatic ring is particularly preferred.

R¹ is a substituent located on a carbon atom constituting the ring A. The number of R¹, that is, "a", may be selected depending on the number of members constituting the aromatic ring A, and for example, may be an integer of 0 to 6, preferably an integer of 0 to 4, and more preferably an integer of 0 to 3. For example, in the case where the ring A is a benzene ring, the number "a" may be an integer of 0 to 3, and in particular an integer of 0 to 2.

The hydrocarbon group represented by R¹ may include an alkyl group (e.g., a linear chain or branched chain C₁₋₁₀alkyl group such as methyl, ethyl, isopropyl, butyl, isobutyl, s-butyl or t-butyl group), a cycloalkyl group (e.g., a C₅₋₈cycloalkyl group such as cyclohexyl group), or an aryl group (e.g., a C₆₋₁₄aryl group such as phenyl or naphthyl group), and others.

R¹ is preferably a hydrocarbon group having a high steric hindrance (or a bulky hydrocarbon group). Such a hydrocarbon group may embrace an alkyl group having a secondary or tertiary carbon atom (a branched chain alkyl group). The alkyl group may have a substituent such as a cycloalkyl group (e.g., a C₅₋₆cycloalkyl group such as cyclohexyl group or an aryl group (e.g., C₆₋₁₀aryl group such as phenyl group).

In the formula (1), the group -OR² is usually a hydroxyl group (that is, R²=H), and may be a derivative group of a hydroxyl group [for example, an alkoxy group (i.e., R² is an alkyl group), and an aliphatic acyl group]. The alkoxy group may include a C₁₋₄alkoxy group such as methoxy group (that is, R² is a C₁₋₄alkyl group such as methyl group), and the aliphatic acyl group may include a C₂₋₅aliphatic acyl group such as acetyl group.

The number of the group -OR², that is, "b" , may be, for example, an integer of 1 to 4, preferably an integer of 1 to 3 and more preferably 1 or 2, depending on the number of members constituting the aromatic ring A.

Examples of the alkylene group represented by R³ may include a linear chain or branched C₁₋₆alkylene such as methylene, ethylene, propylene, trimethylene, 1, 3-butylene or 1,4-butylene, and others. R³ may be a direct bond (that is, in the case of c=0). The preferred R³ includes a direct bond or a linear chain or branched C₁₋₄alkylene group.

The number "d" denotes 0 or 1. When "c" is 0, "d" is 1, and when "c" is 1, "d" may be either 0 or 1.

Incidentally, depending on the numbers "a", "b" and "c", the groups R¹, OR² and R³ may be different independently, respectively.

In the formula (1), the number "e" may be , for example, an integer of 1 to 4, preferably an integer of 1 to 3, and more preferably 1 or 2. Incidentally, in the formula (1), the total number of "e" and "g" may be an integer of 2 to 5.

In the formula (1), X¹ is usually a residue of hydrazine HN-X¹-NH₂ (that is, X¹ represents a direct bond) or residue of a (poly)carboxylic acid X¹-(COOH)ₕ (in the formula, "h" denotes an integer of about 1 to 6). The number "h" is preferably an integer of 1 to 5.

Examples of the organic group represented by X¹ may embrace a hydrocarbon group (e.g., a univalent or polyvalent group corresponding to a hydrocarbon such as an alkane, a cycloalkane, an aromatic ring, or a mono- or polyalkyl-aromatic ring) and a heterocyclic group (e.g., a univalent or polyvalent group corresponding to a 5- to 8-membered heterocycle). X¹ may be a univalent group, and usually, X¹ is preferably a polyvalent (di- to pentavalent) hydrocarbon group, a di- or trivalent 5- or 6-membered heterocyclic group, or the like.

The alkane may include a linear chain or a branched C₁₋₃₄alkane such as methane, ethane, propane, butane, pentane, hexane, heptane, octane, nonane, decane, undecane or dodecane. Examples of the cycloalkane may include a C₅₋₈cycloalkane such as cyclohexane. The aromatic ring may include a bisaryl [for example, a bisaryl having a carbon number of about 12 to 24, e.g., biphenyl and a bisarylalkane (e.g., a bisC₆₋₁₀arylC₁₋₄alkane such as diphenylmethane or 2,2-diphenylpropane)] in addition to a C₆₋₁₄aromatic ring such as benzene or naphthalene. As the mono- or polyalkyl-aromaticring, there may be mentioned the aromatic ring having single or a plurality of alkyl group(s) as a substituent, for example, a mono- to tetraC₁₋₄alkyl-C₆₋₁₀aromatic ring such as xylene, and a C₁₂₋₂₄bisaryl having C₁₋₄alkyl group(s) of about 1 to 4.

Examples of the heterocycle may embrace 5 to 8-membered heterocycle (heterocyclic ring) having, as a hetero atom constituting the ring, at least one member selected from the group consisting of oxygen, sulfur and nitrogen atoms. As the heterocycle, a nitrogen-containing ring such as a triazine ring [e.g., 1,3,5-triazine, 1,2,3-triazine, and 1,2,4-triazine rings (in particular 1,3,5-triazine ring)] or a triazole ring (e.g., 1,2,4-triazole ring) is preferred.

The preferred X¹ may include a direct bond, a polyvalent hydrocarbon group [for example, an aliphatic divalent group such as a linear chain or branched chain C₁₋₃₄alkylene group (e.g., a C₁₋₃₀alkylene group), an aromatic divalent to tetravalent (or bivalent or quadrivalent) group such as an arylene group (in particular, e.g., a di- or trivalent group), and an araliphatic di- or trivalent group (in particular, a divalent group) such as an arylenedialkylene group (e.g., a C₆₋₁₀arylene-diC₁₋₄alkylene group such as xylylene group)] or a di- or trivalent heterocyclic group (e.g., a di- or trivalent 5 to 6-membered nitrogen-containing ring group such as triazin-diyl group), and others. In particular, the preferred X¹ includes a direct bond or a C₁₋₃₄alkylene group (e.g., a C₄₋₂₆alkylene group, particularly a C₆₋₂₂alkylene group), and others.

The carboxylic acid hydrazide (A1) may be produced by a conventional method for producing a carboxylic acid hydrazide, for example, (i) a method of heating an ester (e.g., an alkyl ester) of a hydroxyaryl group-containing carboxylic acid (e.g., a carboxylic acid represented by thefollowingformula (1b)) andahydrazinehydrate (NH₂NH₂), (ii) a method of allowing a halide of the hydroxyaryl group-containing carboxylic acid (e.g., a chloride) to react with hydrazine, (iii) a method of heating a salt of the hydroxyaryl group-containing carboxylic acid and hydrazine, (iv) a method of heating an acid amide corresponding to the hydroxyaryl group-containing carboxylic acid and hydrazine, and (v) a method of allowing an ureide of the hydroxyaryl group-containing carboxylic acid to react with a salt of a hypohalogen acid. Incidentally, in the above-mentioned methods, if necessary, a dicarboxylic acid (e.g., HOOC-X¹-COOH (in the formula, X¹ has the same meaning as defined above)) or a derivative thereof (e.g., a halide, and an alkyl ester) may be used in combination.

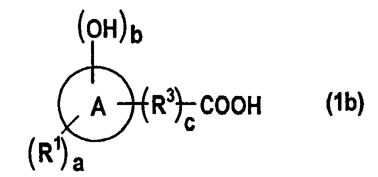

In the formula, R¹, R³, A, "a", "b" and "c" have the same meanings as defined above.

For example, β-(3,5-di-t-butyl-4-hydroxyphenyl)-propionyl-hydrazine [alias name: β-(3,5-di-t-butyl-4-hydroxyphenyl)propionic acid hydrazide] may be obtained, e.g., by a reaction of β-(3,5-di-t-butyl-4-hydroxyphenyl) propionyl chloride or a carboxylic acid ester derivative thereof (e.g., an alkyl ester such as a methyl ester) and a hydrazine hydrate (U.S. Patent No. 3,660,438 specification).

Among the carboxylic acid hydrazides (A1), the preferred compound includes, in the formula (1a), (i) a compound in which R¹ is a linear chain or branched chain C₁₋₆alkyl group, "a" denotes an integer of 0 to 3, "b" denotes an integer of 1 to 3, "c" is 0, "d" is 1, "f" is 1, "g" is 1 or 2, the ring A is a C₆₋₁₀aromatic ring, and X¹ is a linear chain or branched chain C₆₋₂₂alkylene group, and (ii) a compound in which R¹ is a linear chain or branched chain C₁₋₆alkyl group, "a" denotes an integer of 0 to 3, "b" denotes an integer of 1 to 3, "c" is 1, R³ is a linear chain or branched chain C₁₋₄alkylene group, "d" is 0, "f" is 1, "g" is 1, the ring A is a C₆₋₁₀aromatic ring, and X¹ is a direct bond.

### (Non-acrylic polyhydrazide (A2))

The non-acrylic polyhydrazide (A2) comprises a polyhydrazide represented by the following formula (2):

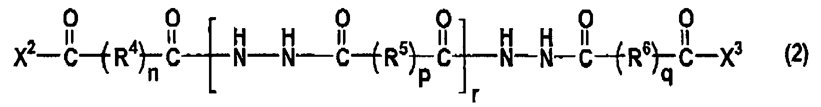

In the formula, R⁴ to R⁶ are the same or different and each represents a divalent hydrocarbon group or heterocyclic group; "n", "p" and "q" are the same or different and each denotes 0 or 1; "r" denotes an integer of not smaller than 0; and X² and X³ are the same or different and each represents a halogen atom, an amino group, a hydrazino group, or a group -OX⁴ (wherein X⁴ represents a hydrogen atom, a hydrocarbon group, an alkali metal atom, or an alkaline earth metal atom); with the proviso that at least any one of X² and X³ is a hydrazino group (-NHNH₂).

The divalent hydrocarbon group represented by R⁴ to R⁶ may include an aliphatic divalent group (e.g., a linear chain or branched chain C₁₋₁₀alkylene group such as methylene, ethylene, propylene, trimethylene, or 1,3- or 1,4-butylene group), an alicyclic divalent group (e.g., a C₅₋₈cycloalkylene group such as cyclohexylene; a divalent group corresponding to a diC₅₋₈cycloalkyl-C₁₋₄alkane such as dicyclohexylmethane; and a C₅₋₈cycloalkylenediC₁₋₆alkylene group such as a hydrogenated xylylene group), an aromatic divalent group (e.g., a C₆₋₁₄arylene group such as phenylene or naphthylene group; biphenylene group; a divalent group corresponding to a bisC₆₋₁₄aryl-C₁₋₆alkane such as diphenylmethane or 2,2-diphenylpropane; and a C₆₋₁₄arylenediC₁₋₆alkylene group such as xylylene group), and others. Among these divalent groups, a linear chain or branched chain C₁₋₆alkylene group (in particular, a C₁₋₄alkylene group), a C₆₋₁₀arylene group, a C₆₋₁₀arylenediC₁₋₄alkylene group, or the like is preferred.

Examples of the heterocyclic group represented by R⁴ to R⁶ may embrace a divalent group corresponding to the heterocycle as exemplified in the paragraph of X¹ in the formula (1).

The number "r" represents the repetition number of the unit. The number "r" may be, for example, about 0 to 100, preferably about 0 to 50 (e.g., about 0 to 30), and more preferably about 0 to 10 (in particular, about 1 to 5). Corresponding to the repetition number "r", the molecular weight of the polyhydrazide (or the number average molecular weight) may be, for example, about 189 to 30,000 (e.g., about 190 to 20,000), preferably about 200 to 15,000, more preferably about 200 to 10,000 (e.g., about 300 to 10,000), and in particular about 250 to 5,000 (e.g., about 250 to 1,000). The polyhydrazide having relatively high molecular weight can inhibit bleeding out even when the polyhydrazide is used in combination with a resin or the like.

The halogen atom represented by X² and X³ may include a fluorine, a chlorine, a bromine and an iodine atoms. Among them, the chlorine or the bromine atom is preferred. In the formula (2), the group -C(=O)OX⁴ represents a carboxyl group (in the case where X⁴ is a hydrogen atom) or a derivative group thereof. Examples of the alkali metal atom represented by X⁴ may embrace a lithium, a potassium and a sodium atoms. As the alkaline earth metal atom, there may be mentioned a magnesium, a calcium and a barium atoms. The hydrocarbon group represented by X⁴ may include a linear chain or branched C₁₋₄alkyl group such as methyl, ethyl or butyl group; a C₅₋₆cycloalkyl group such as cyclohexyl group; a C₆₋₁₀aryl group such as phenyl group; a C₆₋₁₀aryl-C₁₋₄alkyl group such as benzyl group; and others.

In these X² and X³, the preferred combination of X² and X³ includes a combination of a hydrazino group and a hydrazino group, and that of a hydrazino group and an amino group.

Among the polyhydrazides (A2), it is preferred to use a compound in which R⁴ to R⁶ is a linear chain or branched C₁₋₁₀alkylene group, "n"="p"="q"=1, "r" denotes 0 to 5, X² represents a hydrazino group, and X³ represents a hydrazino group or an amino group.

The polyhydrazide (A2) may be obtained by a conventional method, for example, by a reaction of a carboxylic acid hydrazide or hydrazine with a carboxylic acid or a reactive derivative thereof [e.g., a carboxylic acid ester, a carboxylic acid halide, a carboxylic acid amide (in particular, preferably a carboxylic acid ester)]. The number average molecular weight of the polyhydrazide may be controlled by suitably adjusting reaction conditions (e.g. , a molar ratio between the carboxylic acid hydrazide and the carboxylic acid or a reactive derivative thereof). The proportion (molar ratio) of the carboxylic acid hydrazide relative to the carboxylic acid or a reactive derivative thereof may be about 0.7/1 to 3/1, and preferably about 0.8/1 to 2.2/1.

Incidentally, a production method of a high-molecular weight polyhydrazide may be, for example, referred to Japanese Patent Application Laid-Open No. 6535/1980 (JP-55-6535A), U.S. Patent Nos. 2,395,642, 2,615,862 and 3,130,182 specifications, and others.

### (Aminourazole compound (A3))

The aminourazole compound is not particularly limited to a specific one as far as the compound has an urazole ring having an amino group at 4-position, and may embrace various 4-aminourazoles and salts thereof.

Such a 4-aminourazole compound may include, for example, a compound shown in the above-mentioned formula (3).

In the formula (3), the hydrocarbon group represented by R⁷, R⁸ and R⁹ may embrace a hydrocarbon group (e.g., an alkyl group, a cycloalkyl group and an aryl group) and an aralkyl group (e.g., a C₆₋₁₀aryl-C₁₋₄alkyl group such as benzyl group) as exemplified in the paragraph of the above-mentioned R¹.

Among the 4-aminourazole compounds, 4-aminourazole, that is, a compound that all of R⁷, R⁸ and R⁹ in the formula (3) are hydrogen atoms, is preferred.

Incidentally, the 4-aminourazole compound may be, for example, prepared by allowing urea to react with hydrous hydrazine in a polar solvent having a high boiling point. The details regarding the reaction may be, for example, referred to Japanese Patent Application Laid-Open No. 47277/2002 (JP-2002-47277A), and others.

The salt of the 4-aminourazole compound may include a metal salt thereof, a salt thereof with a nitrogen-containing compound. In particular, a metal salt of 4-aminourazole, a salt of 4-aminourazole with a nitrogen-containing compound, and others, are preferred. As the metal forming the metal salt, there may be mentioned, for example, an alkali metal (e.g., K, and Na), an alkaline earth metal (e.g., Mg, Ca, and Ba), a metal of the Group 1B of the Periodic Table of Elements (e.g., Cu, and Ag), a metal of the Group 2B (e.g., Zn), a metal of the Group 3B (e.g., A1), a metal of the Group 4B (e.g., Sn, and Pb), and a metal of the Group 8 (e.g., Fe, Ru, Co, Ni, and Pd). The valence of the metal is not particularly limited to a specific one, and for example, may be 1 to 4, preferably 2 to 4 and more preferably 2 or 3.

Incidentally, the metal salt also embraces, in addition to a salt of the 4-aminourazole compound with the metal (e.g., a univalent to tetravalent metal), an anionic metal salt, for example, a molecular compound comprising the 4-aminourazole compound, a polyvalent metal (e.g., the divalent to tetravalent metal) and an anion (e.g., SO₄²⁻, NO₂⁻, NO₃⁻, Cl⁻, I⁻, ClO₄⁻, and OH⁻).

The nitrogen-containing compound, which forms the salt with the 4-aminourazole compound, may include hydrazine, an acidic nitrogen-containing compound (e.g., an acidic triazine compound such as isocyanuric acid; and an acidic cyclic ureide compound such as barbituric acid, uric acid, acetyleneurea or allantoin), and others.

The salt of the 4-aminourazole compound may be an anhydride (anhydrous salt) or a hydrate.

### [Deodorizing agent composition]

The deodorizing agent of the present invention may further contain a deodorizing auxiliary (such a deodorizing agent is sometimes referred to as a deodorizing agent composition). The combination use of the deodorizing agent and the deodorizing auxiliary ensures to inhibit an aldehyde emission synergistically and to eliminate an aldehyde odor efficiently. In addition, in the case of using the deodorizing agent composition in combination with an aldehyde-generating resin (or an aldehyde-emitting resin), the combination can drastically inhibit emission of an aldehyde from the resin.

As the deodorizing auxiliary, there may be used a basic nitrogen-containing compound (i), a weak acid or a metal salt thereof (ii), a metal compound (iii), an alcohol (iv), a polyphenol (v), an adsorbent inorganic compound (vi), a polyvinyl alcohol, a cyclodextrin, and others. The deodorizing auxiliary may be used singly or in combination. Incidentally, the deodorizing auxiliary may have reactivity to an aldehyde. The deodorizing agent composition includes a composition in which the deodorizing agent is supported to a solid carrier (or support) and/or a clathrate (e.g., adsorbed, intercalated, and enclosed in a clathrate form). The solid carrier (or support) and/or a clathrate may include the adsorbent inorganic compound, the polyvinyl alcohol, and the cyclodextrin, among the deodorizing auxiliaries.
(i) Basic nitrogen-containing compound
   The basic nitrogen-containing compound may embrace a low-molecular weight compound or a high-molecular weight compound (a nitrogen-containing resin), for example, a urea derivative (e.g., a urea compound, an aminotriazine compound, and a guanidine compound), a triazole compound (e.g., a 1,2,4-triazole compound, and a benzotriazole compound), a pyrazolone compound (e.g., a 5-pyrazolone compound), a polyamide, a polyamine, an organic carboxylic acid hydrazide (a carboxylic acid hydrazide other than the above-mentioned specific hydrazine compound (in particular, the carboxylic acid hydrazide)), an amino acid, an amine, an ammonium compound (e.g., an organic carboxylic acid ammonium, and an inorganic ammonium compound), and others. These basic nitrogen-containing compounds may be used singly or in combination.
   Examples of the urea compound may include urea compounds described in Japanese Patent Application Laid-Open No. 212433/2002 (JP-2002-212433A), e.g., a non-cyclic urea compound [for example, urea, an N-substituted urea having a substituent (such as an alkyl group) at N-position, and a non-cyclic urea condensate (e.g., a multimer of urea, such as biuret or biurea; and a condensate of urea and an aldehyde compound, such as methylenediurea or ureaform)], a cyclic urea compound [for example, a cyclic monoureide, e.g., a C₁₋₁₀alkyleneurea (e.g., ethyleneurea, and crotylideneurea), an aryleneurea (e.g., imesatin), a ureide of a dicarboxylic acid (e.g., parabanic acid, barbituric acid, isocyanuric acid, and uramil), a ureide of a β-aldehydic acid (e.g., uracil, thymine, and urazole), and a ureide of an α-hydroxy acid (e.g., a hydantoin compound such as hydantoin, 5,5-dimethylhydantoin or allantoin); and a cyclic diureide, e.g., uric acid, analkyl-substituted uric acid, acetyleneurea (glycoluril), a diureide of an α-hydroxy acid (e.g., 1,1-methylenebis(5,5-dimethylhydantoin)), a diurea such as p-urazine, and a diureide a dicarboxylic acid (e.g., alloxantin, and purpuric acid)].
   The aminotriazine compound may embrace an amino group-containing 1,3,5-triazine compound [for example, melamine or a derivative thereof such as melamine, a substituted melamine (e.g., an alkylmelamine such as 2-methylmelamine, and guanylmelamine), a bismelamine-series compound (e.g., compounds described in WO01/17171 publication or Japanese Patent Application Laid-Open No. 271346/1986 (JP-61-271346A)), a melamine condensate (e.g., melam, melem, and melon), a co-condensed resin of a melamine (e.g. , a melamine-formaldehyde resin, a phenol-melamine resin, a benzoguanamine-melamine resin, and an aromatic polyamine-melamine resin); a cyanuric amide such as ammeline or ammelide; and guanamine or a derivative thereof such as guanamine, acetoguanamine, benzoguanamine, succinoguanamine, adipoguanamine, CTU-guanamine, a polyvinyl-s-triazine, or an imidazoline ring or imidazolyl ring-containing guanamine], an amino group-containing 1,2,3-triazine compound (e.g., 1,2,3-triazine, and benzo-1,2,3-triazine) and a 1,2,4-triazine compound. Incidentally, the arbitrary site constituting a triazine ring (nitrogen atom and carbon atom, particularly carbon atom) may have the amino group(s) as substituent(s). The number of the amino group(s) is not particularly limited to a specific one, and for example, is about 1 to 4, preferably about 1 to 3, and more preferably about 2 to 3. Among the aminotriazines, in particular, the amino group-containing 1,3,5-triazine compound [for example, melamine, the melamine condensate (in particular, e.g., melam, melem, and melon), benzoguanamine, CTU-guanamine, and the imidazolyl ring-containing guanamine] is preferred.
   The guanidine compound may include guanidine compounds described in Japanese Patent Application Laid-Open No. 34417/2000 (JP-2000-34417A), for example, a non-cyclic guanidine (e.g., glycocyamine, guanolin, guanidine, and cyanoguanidine), a cyclic guanidine (e.g., a glycocyamidine compound such as glycocyamidine or creatinine; and oxalylguanidine or an analog thereof (e.g., a cyclic guanidine) such as oxalylguanidine or 2,4-diiminoparabanic acid); an imino group-substituted urazole compound (e.g., iminourazole, and guanazine); an isocyanuric imide (e.g., isoammelide, and isoammeline); malonylguanidine, tartronylguanidine; and mesoxalylguanidine.
   The polyamide may embrace, for example, a homo- or copolyamide such as a polyamide 3 (a polyβ-alanine), a polyamide 46, a polyamide 6, a polyamide 66, a polyamide 11, a polyamide 12, a polyamide MXD6, a polyamide 6-10, a polyamide 6-11, a polyamide 6-12 or a polyamide 6-66-610; a polyamide resin such as a substituted polyamide having a methylol group or an alkoxymethyl group, and a homopolymer or copolymer (which may be a crosslinked polymer) of a poly(meth)acrylamide.
   Examples of the organic carboxylic acid hydrazide may include an aliphatic carboxylic acid hydrazide [e.g., a (poly)hydrazide of a C₂₋₂₄aliphatic (poly)carboxylic acid such as 12-hydroxystearic acid hydrazide, adipic acid dihydrazide, sebacic acid dihydrazide, or dodecane diacid dihydrazide], and an aromatic carboxylic acid hydrazide [e.g., a hydrazide of a C₇₋₁₁ aromatic monocarboxylic acid such as benzoic acid hydrazide, hydroxybenzoic acid hydrazide, or naphthoic acid hydrazide; and a (poly)hydrazide of a C₇₋₁₆ aromatic polycarboxylic acid having no a hydroxyaryl group, such as isophthalic acid dihydrazide, terephthalic acid dihydrazide, benzenetricarboxylic acid trihydrazide, or naphthalenedicarboxylic acid dihydrazide]. Moreover, the organic carboxylic acid hydrazide also includes a N,N'-bis-β-(hydroxyphenyl)alkanoylhydrazine [e.g., compounds described inU.S. Patent No. 3660438 specification, and "Irganox MD 1024" manufactured by Ciba Specialty Chemicals K.K.], an organic carboxylic acid salicyloylhydrazide [e.g., compounds described in U.S. Patent No. 4007183 specification, "ADKSTAB CDA-6" manufactured by Asahi Denka Kogyo K.K., and "MARK CDA-6" manufactured by Adeka Argus Chemical Co., Ltd.].
   The amino acid may embrace a C₂₋₂₀aliphatic amino acid such as an aliphatic amino acid [e.g., glycine, alanine, β-alanine, γ-aminobutyric acid, aminoheptanoic acid, aminononanoic acid, aminoundecanoic acid, hydroxyamino acid (e.g., serine), a sulfur atom-containing amino acid (e.g., cysteine), an acidic amino acid (e.g., asparaginic acid), or a basic amino acid (e.g., lysine, and arginine); and a C₆₋₁₀aryl group-containing C₂₋₂₀aliphatic amino acid such as phenylalanine or tyrosine], an aromatic amino acid [e.g., a C₇₋₁₈aromatic amino acid such as anthranilic acid, or o-, m-, or p-aminobenzoic acid], a heterocyclic amino acid (e.g., proline, hydroxyproline, histidine, and tryptophan) and a derivative of the amino acid [for example, an acidic salt (e.g., a hydrochloride), a metal salt (e.g., an alkali metal salt, an alkaline earth metal salt, and a transition metal salt), an ester derivative, an amide derivative, and a hydrazide derivative). The amino acid may have a plurality of amino groups and/or a plurality of carboxyl groups.
   The amine may include, for example, an aliphatic amine [e.g., a mono- to triC₁₋₄alkylamine such as diethylamine or triethylamine; and a mono- to tris(hydroxyC₁₋₄alkyl)amine such as methanolamine, tris(hydroxymethyl)amine, ethanolamine or diethanolamine], an aromatic amine (e.g., an aromatic secondary amine or tertiary amine such as o-, m- or p-toluidine, or o-, m- or p-phenylenediamine), and a polyamine (e.g., a polyethyleneimine, a polyvinylamine, a polyallylamine or a copolymer thereof).
   As the ammonium compound, for example, ammonium compounds described in WO01/62309 publication may be used.
   Examples of the organic carboxylic acid ammonium may embrace an ammonium salt of an organic carboxylic acid, e.g., an ammonium salt of a saturated aliphatic carboxylic acid [e.g., a saturated C₁₋₃₄monocarboxylic acid such as acetic acid, propionic acid, butyric acid or valeric acid; a saturated C₂₋₃₀dicarboxylic acid such as succinic acid, adipic acid or sebacic acid; and a hydroxy acid corresponding thereto (e.g., lactic acid, hydroxybutyric acid, 12-hydroxystearic acid, tartaric acid, and citric acid)] or an unsaturated aliphatic carboxylic acid [for example, an unsaturated C₃₋₃₄monocarboxylic acid such as (meth)acrylic acid or crotonic acid; an unsaturated C₄₋₃₀dicarboxylic acid such as maleic acid or fumaric acid; and a hydroxy acid corresponding thereto (e.g., propiolic acid)].
   The inorganic ammonium compound may include an ammonium salt of an inorganic acid, for example, an ammonium halide (e.g., ammonium fluoride, ammonium chloride, ammonium bromide, and ammonium iodide), an ammonium sulfate [for example, an ammonium salt of various sulfuric acids (e.g., a non-condensed sulfuric acid such as peroxomonosulfuric acid, sulfuric acid or sulfurous acid; and a condensed sulfuric acid such as peroxodisulfuric acid or pyrosulfuric acid)], an ammonium (poly)phosphate, and ammonium carbonate. Among the inorganic ammonium compounds, ammonium chloride or ammonium sulfate is preferred.
(ii) Weak acid or a metal salt thereof
   The weak acid may embrace the organic carboxylic acid exemplified in the paragraph of the organic carboxylic acid ammonium salt, a boric acid (e.g., a non-condensed boric acid such as orthoboric acid, and a condensed boric acid such as tetraboric acid or boric anhydride), and carbonic acid.
   The metal salt of the weak acid may include, for example, metal salts of weak acids described in WO01/62309 publication. As the metal salt of the weak acid, there may be mentioned, for example, a salt thereof with an alkali metal (e.g., K, and Na), an alkaline earth metal (e.g., Mg, and Ca), or a transition metal [for example, a metal of the Group 3A (e.g., Sc), a metal of the Group 4A (e.g., Ti, and Zr), a metal of the Group 5A (e.g., V), a metal of the Group 6A (e.g., Mo, and W), a metal of the Group 7A (e.g., Mn), a metal of the Group 8 (e.g., Fe, and Ni), and a metal of the Group 1B (e.g., Cu) of the Periodic Table of Elements]. Among metal salts of the weak acids, the alkali metal salt or the alkaline earth metal salt is preferred, and in particular, the alkali metal salt of the organic carboxylic acid (e.g., potassium acetate, sodium acetate, sodium stearate, and sodium 12-hydroxystearate), the alkaline earth metal salt of the organic carboxylic acid (e.g., calcium acetate, magnesium stearate, calcium stearate, calcium 12-hydroxystearate, calcium tartrate, and calcium citrate).
   These weak acids or metal salts thereof may be used singly or in combination.
(iii) Metal compound
   The metal compound may embrace a metal oxide, a hydrate of a metal oxide (e.g., metal hydroxide), and a metal chloride. The metal compound may be either an anhydride or a hydrate. Incidentally, in present description, a (hydrous) metal oxide is sometimes a general term for the metal oxide and the metal hydroxide.
   Examples of the metal constituting the metal compound may include the metal exemplified in the paragraph of the aminourazole compound, the metal described in the paragraph of the metal salt of the weak acid, and a metal of the Group 4B of the Periodic Table of Elements (e.g., Sn). The metal compound may be a complex metal compound having a plurality of these metals (e.g., a complex metal oxide, and a complex metal chloride). Among these metals, the alkaline earth metal is particularly preferred.
   The concrete examples of the alkaline earth metal compound may embrace an oxide which may be a hydrous oxide (e.g., magnesium oxide, and magnesium hydroxide), and a chloride (e.g., calcium chloride).
   These metal compounds may be used alone or in combination.
(iv) Alcohol
   The alcohol may include a monoalcohol (e.g., a saturated or unsaturated C₄₋₂₄aliphatic alcohol such as myristyl alcohol, stearyl alcohol or oleyl alcohol; and a saturated or unsaturated C₅₋₈alicyclic alcohol such as cyclohexyl alcohol), and usually, it is preferred to employ a polyhydric alcohol as the alcohol. Examples of the polyhydric alcohol may embrace a diol [e.g., a C₂₋₁₂alkylene glycol such as ethylene glycol, trimethylene glycol, propylene glycol, 1,4-butanediol, 1,3-butanediol, neopentyl glycol, hexanediol, octanediol or decanediol; a polyoxyC₂-₄alkylene glycol (e.g., diethylene glycol, triethylene glycol, a polyethylene glycol, dipropylene glycol, tripropylene glycol, and a polytetramethylene glycol); and an alicyclic diol (e.g., 1,4-cyclohexanediol, 1,4-cyclohexanedimethanol, and hydrogenated bisphenol A)], and in addition, a polyhydric alcohol such as glycerin, diglycerin, trimethylolpropane, trimethylolethane, pentaerythritol, dipentaerythritol, a polypentaerythritol, sorbitol, (α-, β-, γ-, δ-, or ε-) cyclodextrin, a trehalose, inositol, maltose, chitin, chitosan, a polyvinyl alcohol or a copolymer thereof, or a poly(2-hydroxyethyl (meth)acrylate) or a copolymer thereof. The alcohols may be used singly or in combination.
   Among these alcohols, the particularly preferred one includes the polyalkylene glycol such as diethylene glycol; and the polyhydric alcohol such as glycerin, a (poly)pentaerythritol, sorbitol, a cyclodextrin, a trehalose or a polyvinyl alcohol.
(v) Polyphenol
   The polyphenol may embrace, for example, a polyhydric phenol compound, a bisphenol compound, a calixarene, a catechin compound (e.g., catechin, epicatechin, gallocatechin, epigallocatechin, epicatechingallate, epigallocatechingallate, quercetin, caempherol, and myricetin), a teanine compound, a tannin compound and a lignin compound. The catechin compound is, for example, available as "SUNFLAVON series (e.g., HG, P)" from Taiyo Kagaku Co., Ltd.
   Examples of the polyhydric phenol compound may include a dihydric phenol such as catechol, resorcin, hydroquinone or 2,6-dihydroxynaphthalene; a trihydric phenol such as pyrogallol or phloroglucin; phenollignin, and gallic acid.
   These polyphenol compounds may be used alone or in combination.
(vi) Adsorbent inorganic compound
   The adsorbent inorganic compound may include an inorganic compound known as a porous carrier (or support). Examples of such an adsorbent inorganic compound may embrace a stratiform or porous inorganic oxide (for example, an aluminum and/or silicon oxide, e.g., a hydrotalcite, a zeolite, a silica gel, an alumina, a boehmite, a sepiolite, and a metal salt of phosphoric acid (e.g., zirconium phosphate, and aluminum phosphate)), a pearlite, and an activated carbon (e.g., an activated carbon derived from a mineral). The zeolite may be a synthetic zeolite or a natural zeolite (e.g., faujasite). These adsorbent inorganic compounds may be used alone or in combination.

The proportion of the deodorizing auxiliary may be, for example, selected from the wide range depending on the species thereof, e.g., from about 0.01 to 10000 parts by weight relative to 100 parts by weight of the deodorizing agent. The proportion may be, for example, about 0.01 to 5000 parts by weight (e.g., about 0.01 to 3000 parts by weight), preferably about 0.05 to 2000 parts by weight (e. g. , about 0.1 to 1500 parts by weight), and more preferably about 0.5 to 1500 parts by weight (e.g., about 1 to 1000 parts by weight), relative to 100 parts by weight of the deodorizing agent. Moreover, the proportion may be, for example, about 0.01 to 100 parts by weight, preferably about 0.01 to 50 parts by weight (e.g., about 0.05 to 30 parts by weight), more preferably about 0.1 to 25 parts by weight (e.g., about 1 to 20 parts by weight), in particular, about 2 to 15 parts by weight. Incidentally, in the case where the deodorizing agent and the deodorizing auxiliary capable of carrying (or supporting) the deodorizing agent (e.g., the adsorbent inorganic compound, the cyclodextrin, and the polyvinyl alcohol) are used in combination, the proportion may be about 30 to 10000 parts by weight, preferably about 50 to 5000 parts by weight, and more preferably about 100 to 1000 parts by weight.

### [Resin composition]

The resin composition of the present invention (including the odor-eliminating resin composition) comprises a resin and at least the hydrazine compound, and may comprise the resin and the deodorizing agent (or the deodorizing agent composition). The combination use of the hydrazine compound and the resin ensures to provide a resin composition having an excellent odor-eliminating property. Moreover, in the case of using an aldehyde-generating resin (e.g., a polyacetal resin, and an amino resin) as the resin, the composition allows to remarkably reduce the amount of an aldehyde emitted from the resin. Further, in the resin composition of the present invention, a pellet of the resin (e.g., a pellet of a polyacetal resin) may coexist in a master batch containing the hydrazine compound (or the deodorizing agent or the deodorizing agent composition).

### (Resin)

The resin may be any of a natural resin or a synthetic resin, and may be any of a thermosetting resin or a thermoplastic resin.

The natural resin may include, for example, a rosin, a shellac, a dammar, a starch, and a derivative thereof. In the synthetic resin, examples of the thermosetting resin may embrace a diallyl phthalate resin, a vinyl ester resin, an unsaturated polyester resin, a maleic acid resin, an epoxy resin, a polyurethane resin, a polyimide resin, a phenolic resin, an amino resin (e.g., a urea resin, a melamine resin, and a guanamine resin), and a silicon resin.

The preferred resin may include the thermoplastic resin, e.g., an olefinic resin, a halogen-containing vinyl-series resin, a styrenic resin, an acrylic resin, a polyester-series resin, a polyamide-series resin, a polyurethane-series resin, a polyacetal-series resin, a polyether resin [for example, a polyphenylene oxide-series resin, and a polyalkylene oxide (e.g., a polyC₂₋₆alkylene oxide such as a polyethylene oxide)], a polyphenylene sulfide-series resin, a polycarbonate-series resin, a polyvinyl alcohol, a polyvinyl acetate, a polyvinyl acetal, a polyamideimide, a polyimide, a polyetherimide, a polysulfone (e.g., a thermoplastic polysulfone, a poly(ethersulfone), and a poly(4,4'-bisphenolethersulfone), a polyetheretherketone, a polyetherketone, a cellulose derivative (e.g., a cellulose ester, a celluloid), an elastomer (e.g., a polyurethane elastomer) or a rubber (e.g., a natural rubber, and a hydrochloric rubber), and a copolymer containing a constituent component of these polymers as a constitutional unit. These resins may be used singly or in combination.

Incidentally, the deodorizing agent and the deodorizing agent composition of the present invention, being excellent in aldehyde-trapping effects, can efficiently inhibit an aldehyde emission from a resin which is cable of emitting (or generating) an aldehyde due to an action by heat, light, oxygen, an acid or alkali, water or others even in the case of the combination use of the agent or composition and the resin. Further, even in the case where an aldehyde is generated from the resin, the deodorizing agent and the deodorizing agent composition can eliminate the odor of the aldehyde effectively. The aldehyde-generating resin may include a polyacetal-series resin (e.g., a polyacetal homopolymer or copolymer), a polyvinyl acetal (e.g., a polyvinyl formal, a polyvinyl butyral), a phenolic resin (e.g., a novolak or resol phenolic resin), a furan resin, the above-mentioned amino resin (e.g., an aminoaldehyde-series resin), and others.

The resin preferably includes a resin having formability or moldability (e.g., a thermoforming property such as a film-forming performance or an injection molding property), and may have not only a linear chain structure but also a branched chain structure and/or a crosslinked structure. Moreover, in the resin, the degree of polymerization, the degree of branch, the state of copolymerization (e.g., a random, a block, and a graft) and the compositional proportion of copolymer are not particularly limited to specific ones.

Among the thermoplastic resins, the olefinic resin may embrace, for example, a homo- or copolymer of an α-olefin (such as ethylene, propylene or 4-methylpentene) (e.g., a polyethylene, a polypropylene, and an ethylene-propylene copolymer); a copolymer of the α-olefin and a copolymerizable monomer (e.g., a vinyl halide compound such as vinyl chloride, vinyl acetate, and a acrylic monomer), or a derivative of the copolymer (e.g., an ethylene-vinyl acetate copolymer, and an ethylene-ethyl acrylate copolymer); and a halogen-containing olefinic resin (e.g., a chlorinated polyethylene).

The halogen-containing vinyl-series resin may include, for example, a homo- or copolymer of a halogen-containing vinyl monomer such as vinyl chloride or vinylidene chloride (e.g., a polyvinyl chloride, a polyvinylidene chloride, and a vinyl chloride-vinylidene chloride copolymer); and a copolymer of a halogen-containing vinyl monomer and a copolymerizable monomer [e.g., a vinylic monomer such as vinyl acetate, a styrenic monomer, an acrylic monomer or maleic anhydride; and a diene-series monomer such as butadiene or isoprene (e.g., a vinyl chloride -vinyl acetate copolymer, a vinyl chloride-styrene copolymer, a vinyl chloride-acrylonitrile copolymer, and a vinyl chloride-(meth)acrylic ester copolymer).

Examples of the styrenic resin may include a homo- or copolymer of an aromatic vinyl monomer (e.g., styrene, and α-methylstyrene) (for example, a polystyrene); a copolymer of an aromatic vinyl monomer and a copolymerizable monomer [e.g., an acrylic monomer such as (meth)acrylonitrile or (meth)acrylic ester, and maleic anhydride] [for example, an acrylic monomer-styrene copolymer such as an acrylonitrile-styrene copolymer (an AS resin) or a (meth)acrylate-styrene copolymer]; and a rubber-modified styrene-series copolymer [for example, a copolymer of styrene and an elastomer (such as a butadiene rubber or an acrylic rubber) or maleic acid, e.g., an acrylonitrile-butadiene-styrene copolymer (an ABS resin)].

The acrylic resin may embrace, for example, a poly(meth)acrylonitrile, and a poly(meth)acrylic ester [e.g., a poly(C₁₋₁₀alkyl (meth)acrylate) such as a poly(methyl methacrylate) or a poly(ethyl acrylate)].

Examples of the polyester-series resin may include a homopolyester such as a polyC₂₋₆alkylene terephthalate (e.g., a polyethylene terephthalate, and a polybutylene terephthalate) or a polyC₂₋₆alkylene naphthalate (e.g., a polyethylene naphthalate, and a polybutylene naphthalate); a copolyester having a C₂₋₆alkylene terephthalate or a C₂₋₆alkylene naphthalate as a main repeating unit; and a polyarylate and a polyester elastomer.

The amide-series resin may embrace, for example, a polyamide such as a polyamide 6, a polyamide 66 , a polyamide 11, a polyamide 12, a polyamide 46, a polyamide 610 or a polyamide 612, and a polyamide elastomer. The polyurethane-series resin may include, for example, a polyurethane, and a polyurethane elastomer.

The polyacetal resin may include a high molecular compound containing oxymethylene group (-OCH₂-) as a predominant constituent unit, for example, a polyacetal homopolymer or polyoxymethylene (e.g., trade name "Delrin" manufactured by DuPont, U.S.A.; trade name "Tenac 4010" manufactured by Asahi Kasei Corp.), and a polyacetal copolymer containing an oxymethylene unit and a comonomer unit (e.g., trade name "Duracon" manufactured by Polyplastics Co., Ltd.). Referring to such a copolymer, the comonomer unit embraces an oxyalkylene unit of about 2 to 6 carbon atoms (preferably about 2 to 4 carbon atoms), for example, oxyethylene group (-OCH₂CH₂-), oxypropylene group, and oxytetramethylene group. The content of the comonomer unit may be small, and may be selected from the range of about 0.01 to 20 mol%, preferably about 0.03 to 15 mol% (e.g., about 0.05 to 10 mol%), and more preferably about 0.1 to 10 mol%, relative to the whole polyacetal resin.

The polyacetal copolymer may include a copolymer containing two components, a terpolymer containing three components, and others. The polyacetal copolymer may also be a random copolymer, and in addition, a block copolymer [e.g., Japanese Patent Publication No. 24307/1990 (JP-2-24307B), and trade names "Tenac LA" and "Tenac LM" manufactured by Asahi Kasei Corp.], or a graft copolymer. Moreover, the polyacetal resin may have a linear (or straight) structure or branched structure, and may have a crosslinked structure. In addition, the end groups of the polyacetal resin may be stabilized by esterification with a carboxylic acid such as acetic acid or propionic acid, or an anhydride thereof, urethanation with an isocyanate compound, or etherification. Concerning the polyacetal resin, there is no particular limitation on the degree of polymerization, the degree of branching, or the degree of crosslinking, only provided it can be melt-molded. There is no particular restriction as to the molecular weight of the polyacetal resin, and for example, the weight average molecular weight is about 5,000 to 500,000 and preferably about 10,000 to 400,000.

The polyacetal resin may be produced, for example, by homo- or copolymerizing an aldehyde (e. g. , formaldehyde, paraformaldehyde, and acetaldehyde), and/or a component such as trioxane or tetraoxane, or by copolymerizing the above-mentioned component and other cyclic ether (e.g., ethylene oxide, propylene oxide, butylene oxide, styrene oxide, cyclohexane oxide, cyclohexene oxide, 1,3-dioxolane, and 1,3-dioxane) and/or a cyclic formal (e.g., diethylene glycol formal, and 1,4-butanediol formal). Further, as the copolymerizable component, an alkyl or aryl glycidyl ether (e.g., methyl glycidyl ether, ethyl glycidyl ether, phenyl glycidyl ether, and naphthyl glycidyl ether), an alkylene or polyalkylene glycol diglycidyl ether (e.g., ethylene glycol diglycidyl ether, triethylene glycol diglycidyl ether, and butanediol diglycidyl ether), an alkyl or aryl glycidyl alcohol, a cyclic ester (e.g., β-propiolactone), and/or a vinyl compound (e.g., styrene, vinyl ether) may be employed.

Among the resins, the thermoplastic resin (e.g., the polyacetal-series resin, the olefinic resin, the halogen-containing vinyl-series resin, the styrenic resin, the acrylic resin, the polyester-series resin, and the polyurethane-series resin) is preferred.

The resin may be used in the form of a solid, or in the form of a solution (e.g., an organic solvent solution, and an aqueous solution) or a dispersion liquid (e.g., an aqueous emulsion).

The deodorizing agent (the hydrazine compound) and the deodorizing agent composition of the present invention can effectively trap an aldehyde (e.g., formaldehyde) to eliminate an aldehyde odor even when the amount of the agent (or the composition) is small. In addition, the agent and the composition have a high affinity to the resin. The agent and the composition of the present invention, therefore, prevent any loss of the properties of the resin composition, can inhibit a bleeding out from the resin, and can maintain an aldehyde-trapping performance or odor-eliminating performance for a long period of time.

The resin composition may comprise the deodorizing agent (or the hydrazine compound) at an effective amount for expressing a trapping performance to an aldehyde (e.g., formaldehyde). The proportion of the agent may be, for example, about 0.001 to 30 parts by weight (e.g., about 0.001 to 25 parts by weight), preferably about 0.002 to 15 parts by weight, more preferably about 0.005 to 10 parts by weight, and may be about 0.01 to 5 parts by weight (e.g., about 0.01 to 3 parts by weight), relative to 100 parts by weight of the resin, and in such a proportion, the agent can effectively inhibit the aldehyde odor.

In the case where the resin composition comprises the deodorizing auxiliary, the proportion of the deodorizing auxiliary may be effective for exhibiting a trapping performance to an aldehyde (e.g., formaldehyde), and may be, for example, about 0.0001 to 10 parts by weight (e.g., about 0.001 to 10 parts by weight), preferably about 0.005 to 5 parts by weight, more preferably about 0.01 to 5 parts by weight, and may be about 0.01 to 3 parts by weight (e.g., about 0.01 to 1 part by weight), relative to 100 parts by weight of the resin.

The resin composition may further contain a foaming agent, a stabilizer [for example, an antioxidant (e.g., a hindered phenol, and a hindered amine), an ultraviolet ray absorbing agent, a heat stabilizer (e.g., a basic nitrogen compound such as an aminotriazine compound, a guanidine compound, a urea compound, an aminoalcohol compound or an imide compound; a salt of an inorganic acid with an alkali or alkaline earth metal; a zeolite; and a phosphine compound), a processing stabilizer (for example, a long chain (or highly) fatty acid (e.g., a saturated C₁₀₋₂₆aliphatic mono- or dicarboxylic acid, and an unsaturated C₁₀₋₂₀aliphatic mono- or dicarboxylic acid) or a derivative thereof (e.g., an acid amide with an amine such as a monoamine or a diamine, and an ester with an aliphatic mono- or polyol), a polyalkylene glycol (for example, a polyC₂₋₆alkylene glycol (e.g., a polyC₂₋₄alkylene glycol having a number average molecular weight of about 3 x 10² to 1 x 10⁶), and a silicone compound (e.g., a monoorganosiloxane such as a diC₁₋₆alkylsiloxane, a C₁₋₄alkylC₆₋₁₀arylsiloxane or a diC₆₋₁₀arylsiloxane; and a homopolymer or copolymer of a monoorganosiloxane)), and a weather (light)-resistant stabilizer (e.g., a benzophenone-series compound, an aromatic benzoate-series compound, a cyanoacrylate-series compound, an oxalic anilide-series compound, and a hydroxyphenyl-1,3,5-triazine-series compound)], an impact resistance improver [for example, an acrylic core-shell polymer, a polyurethane-series resin, and a polyester-series resin], a gloss-controlling agent [for example, an acrylic resin (e.g., a homo- or copolymer of a C₁₋₁₀alkyl (meth)acrylate such as a poly(methyl methacrylate), an AS resin, and an AES resin) and a styrenic resin (e.g. , a homo- or copolymer of styrene), in addition to the resin as exemplified in the paragraph of the impact resistance improver], a slip-improving agent [e.g., an olefinic resin, a silicone-series resin, and a fluorine-series resin], a plasticizer, a reinforcing agent , a filler [for example, a fibrous filler (e.g., an inorganic fiber such as a glass fiber, a carbon fiber or a whisker; and an organic fiber such as an amide fiber), a plate-like filler (e.g., a glass flake, a mica, and a variety of metal foil), and a particulate filler (e.g., a glass such as a milled fiber or a glass bead; a metal oxide such as zinc oxide or an alumina; a sulfate such as calcium sulfate; a carbonate such as calcium carbonate; a silicate such as a talc; a sulfide such as molybdenum disulfide; and a carbide such as graphite fluoride or silicon carbide)], an antistatic agent, a surfactant, a flame retardant, a lubricant, a coloring agent [for example, a dye (e.g., an organic dye such as a solvent-series dye), and an inorganic or organic pigment], a fungicide, and an antibacterial agent. These additives may be used alone or in combination.

Among the antioxidants, the hindered phenol may include, for example, a C₂₋₁₀alkylene glycol bis[3-(3,5-di-t-butyl-4-hydroxyphenyl)propionatel; a di or trioxyC₂₋₄alkylene glycol-bis[3-(3,5-di-t-butyl-4-hydroxyphenyl)propionate]; e.g., a di or trioxyC₂₋₄alkylene glycol-bis[3-(3-t-butyl-5-methyl-4-hydroxyphenyl)propionate] such as triethylene glycol-bis[3-(3-t-butyl-5-methyl-4-hydroxyphenyl)propionate]; a C₃₋₈alkanetriol-bis[3-(3,5-di-t-butyl-4-hydroxyphenyl)propionate]; e.g., a C₄₋₈alkanetetraol tetrakis[3-(3,5-di-t-butyl-4-hydroxyphenyl)propionate] such as pentaerythritol tetrakis[3-(3,5-di-t-butyl-4-hydroxyphenyl)propionate]. Moreover, the hindered amine may embrace, for example, a tri- or tetraC₁₋₃alkylpiperidine or a derivative thereof, a bis(tri-, tetra- or pentaC₁₋₃alkylpiperidiyl) C₂₋₂₀alkanedicarboxylate [for example, bis(2,2,6,6-tetramethyl-4-piperidyl)oxalate], bis(2,2,6,6-tetramethyl-4-piperidyl)sebacate, bis(1,2,2,6,6-pentamethyl-4-piperidyl)sebacate, 1,2-bis(2,2,6,6-tetramethyl-4-piperidyloxy)ethane, phenylnaphthylamine, 4,4'-bis(α,α-dimethylbenzyl)diphenylamine, N,N'-diphenyl-1,4-phenylenediamine, and N-phenyl-N'-cyclohexyl-1,4-phenylenediamine.

Among these additives, usually, at least one member selected from the group consisting of the antioxidant, the heat stabilizer, the processing stabilizer, the weather (light)-resistant stabilizer, the impact resistance improver, the gloss-controlling agent, the slip-improving agent, the coloring agent and the filler may be employed. Moreover, each additive may be used alone or in combination. The above-mentioned additive(s) can not only impart each function of the additive(s) to the resin or the resin composition but also, if anything, synergistically improve the inhibitory action of the specific hydrazine compound against the formaldehyde emission by the combined use with the hydrazine compound without deterioration of the inhibitory action. Incidentally, the addition of the antioxidant and/or the heat stabilizer to the resin (e.g., the polyacetal resin) ensures to impart a long-term stability or a heat resistance to the resin. Moreover, the addition of the processing stabilizer to the resin can impart a formability (e.g., a mold releasing property, and a reduction of mold deposit) to the resin.

In the case where the resin composition comprises the above-mentioned additive(s), the proportion of each additive may be selected from the following range. The proportion of the antioxidant may be about 0.001 to 5 parts by weight, and preferably about 0.005 to 3 parts by weight (e.g., about 0.01 to 2 parts by weight), relative to 100 parts by weight of the resin. The proportion of the heat stabilizer relative to 100 parts by weight of the resin may be, for example, about 0.001 to 10 parts by weight, and preferably about 0.001 to 5 parts by weight (in particular, about 0.01 to 2 parts by weight). The proportion of the processing stabilizer may be, for example, about 0.001 to 10 parts by weight, preferably about 0.01 to 5 parts by weight, and more preferably about 0.03 to 3 parts by weight, relative to 100 parts by weight of the resin. The proportion of the weather (light)-resistant stabilizer relative to 100 parts by weight of the resin may be, for example, about 0.01 to 5 parts by weight, preferably about 0.1 to 4 parts by weight, and more preferably about 0.1 to 2 parts by weight. The proportion of the impact resistance improver may be, for example, about 0.1 to 30 parts by weight, preferably about 0.5 to 25 parts by weight, and more preferably about 1 to 20 parts by weight, relative to 100 parts by weight of the resin. The proportion of the gloss-controlling agent relative to 100 parts by weight of the resin may be, for example, about 0.01 to 30 parts by weight, preferably about 0.02 to 10 parts by weight, and more preferably about 0.05 to 5 parts by weight. The proportion of the slip-improving agent may be, for example, about 0. 1 to 50 parts by weight, preferably about 1 to 30 parts by weight, and more preferably about 3 to 20 parts by weight, relative to 100 parts by weight of the resin. The content of the coloring agent may be, for example, about 0.01 to 5 parts by weight, preferably about 0.1 to 4 parts by weight, and more preferably about 0.2 to 2 parts by weight, relative to 100 parts by weight of the resin. The proportion of the filler relative to 100 parts by weight of the resin may be, for example, about 1 to 100 parts by weight, preferably about 3 to 80 parts by weight, and more preferably about 5 to 50 parts by weight.

The resin composition of the present invention may be a particulate mixture or a molten mixture, and may be prepared by mixing the resin (e.g., the polyacetal resin) and the deodorizing agent (or the hydrazine compound), and if necessary, the deodorizing auxiliary or other additive [for example, the stabilizer (e.g., the antioxidant, the processing stabilizer, the heat stabilizer, and the weather (light)-resistant stabilizer), the impact resistance improver, the gloss-controlling agent, the slip-improving agent, the coloring agent and/or the filler] in a conventional method.

For example, the following method may be adopted: (1) a method comprising feeding (or supplying) all components through a main feed port (or a main supply port) of an extruder (e.g., a uniaxial or biaxial extruder), kneading and extruding the fed components to prepare pellets, and molding the pellets; (2) a method comprising feeding component(s) having no specific hydrazine compound mentioned above (e.g., the resin such as the polyacetal resin, the deodorizing auxiliary, and other additive(s)) through a main feed port of an extruder and feeding component(s) containing at least the specific hydrazine compound (or the deodorizing agent) (e.g., the resin such as the polyacetal resin, the deodorizing auxiliary and/or other additive(s) mentioned above in addition to the hydrazine compound) from a side feed port, kneading and extruding the fed components to prepare pellets, and molding the pellets; (3) a method comprising feeding component (s) containing part of the specific hydrazine compound (or the deodorizing agent) (e.g., the resin such as the polyacetal resin, the deodorizing auxiliary and/or other additive(s) in addition to the hydrazine compound) through a main feed port and feeding component(s) containing the residual specific hydrazine compound (or the deodorizing agent) (e.g., the resin such as the polyacetal resin, the deodorizing auxiliary and/or other additive(s) in addition to the hydrazine compound) through a side feed port of an extruder, kneading and extruding the fed components to prepare pellets, and molding the pellets; (4) a method comprising once making pellets (master batch) different in formulation from the aimed article, mixing (diluting) the pellets in a certain proportion with other component(s), and molding the resulting pellets to give a shaped article having a predetermined formulation; and (5) a method comprising allowing the specific hydrazine compound (or the deodorizing agent) to coexist with (or attach to) a pellet of the resin (e.g., the polyacetal resin) by spraying, coating (e.g., surface-coating) or other means, and then molding the resulting matter into a shaped article having a predetermined formulation.

Among these methods, the methods (1), (2), (3) and (5) are preferred. In particular, it is preferred to melt-mix (melt-knead) components to prepare the polyacetal resin composition by using a uniaxial or biaxial extruder having exhaust vent port(s) of not less than 1. Moreover, the heterocyclic compound may be fed through the feed port located in either the upstream or the downstream of the exhaust vent port. Further, in the extruding and preparing step, the amount of formaldehyde emitted from the obtained shaped (or molded) article can be further reduced by preblending a processing auxiliary such as water and/or an alcohol (e.g., methanol, ethanol, isopropyl alcohol, and n-propyl alcohol), or injecting the processing auxiliary through a feed port of the upstream of an exhaust vent port, and removing by exhausting volatile component(s) containing water and/or the alcohol through the exhaust vent port. The amount of water and/or the alcohol as the processing auxiliary is not particularly limited to a specific one, and may be usually selected from the range of about 0 to 20 parts by weight, preferably about 0.01 to 10 parts by weight, and more preferably about 0.1 to 5 parts by weight, relative to 100 parts by weight of the resin (e.g., the polyacetal resin).

Moreover, the hydrazine compound has a high formaldehyde-trapping rate, meanwhile limits a trapping amount of formaldehyde. In particular, in the case of melt-mixing the polyacetal resin and the hydrazine compound by using an extruder, therefore, the preferably used method is an extruding and preparing method comprising side-feeding part or all of at least the hydrazine compound through a side feed port of the extruder, and/or an extruding and preparing method comprising setting up a melt-kneading or melt-mixing time (residence time of the components) in the extruder as a short time, e.g., not longer than 300 seconds (e.g., about 5 to 300 seconds), preferably not longer than 250 seconds (e.g., about 10 to 250 seconds), more preferably not longer than 200 seconds (e.g., about 10 to 200 seconds), and particularly about 10 to 150 seconds.

Incidentally, in the preparation of a composition for use in a shaped article, mixing of a particulate of a resin (e.g., a polyacetal resin) [e.g., a particulate (powder) obtained by milling or grinding part or all of the resin] as a base (or substrate) with other components [e.g., the specific hydrazine compound (or the deodorizing agent or the deodorizing agent composition), the deodorizing auxiliary, other additive(s) (e.g., the stabilizer, the impact resistance improver, the slip-improving agent, the coloring agent and/or the filler)] followed with melt-kneading advantageously improves the degree of dispersion of the additives.

The polyacetal resin composition of the present invention realizes that the emission of formaldehyde due to oxidation or thermal decomposition or the like of the polyacetal resin is remarkably restrained or inhibited particularly in the molding and processing (particularly, a melt-molding and processing) step and that the working environment is improved or ameliorated. Moreover, deposition of decomposition products or additives on a mold (mold deposit), blooming or bleeding of such decomposition products or additives from a shaped article can be remarkably restricted or inhibited, and various problems on the molding and processing step can be overcome.

Since the resin composition of the present invention (e.g., the deodorizing resin composition) can efficiently reduce an aldehyde odor, the resin composition is useful for a shaped article of the deodorizing resin. That is, the present invention also include a shaped article formed from the resin composition (or deodorizing resin composition) comprising the hydrazine compound (or the deodorizing agent, or the deodorizing agent composition) and the resin.

The deodorizing agent (or the deodorizing agent composition), the resin composition (e.g., the deodorizing resin composition) or the shaped article formed from the resin composition (e.g., the deodorizing resin composition) of the present invention can efficiently eliminate an aldehyde odor (or trap an aldehyde) by (1) disposition close to an aldehyde-generating source (e.g., contact, coating, or packaging), or (2) inclusion in the aldehyde-generating source.

Incidentally, the species of the aldehyde-generating source is not particularly limited to a specific one, and for example, aldehyde-generating building materials, furniture, aldehyde odor-emitting components (e.g., a tobacco, and an aldehyde-generating resin). The deodorizing agent, the deodorizing agent composition and the resin composition may be, for example, in the form of a solution, a dispersion liquid or a particulate, depending on application or mode of usage. Incidentally, the resin composition having a solution or dispersion liquid form may be a paint and varnish, a print ink, a coating material (or agent), an adhesive, a sizing agent, and others.

In the above-mentioned method (1), (i) the deodorizing agent (the deodorizing agent composition or the deodorizing resin composition) may be disposed alone in the neighborhood of the aldehyde-generating source, or (ii) a deodorizing shaped article formed by applying the deodorizing agent to a substrate (including a shaped article) may be disposed in close vicinity to the aldehyde-generating source.

In the embodiment (i), for example, the deodorizing agent-containing solution (or dispersion liquid) may be coated or applied on at least part of the surface of the aldehyde-generating source, or the solution may be impregnated with the aldehyde-generatingsource. Moreover, a deodorizing layer containing the deodorizing agent may be laminated on the aldehyde-generating source by a lamination method or other means. The shaped article formed from the deodorizing resin composition may be disposed in the neighborhood of the aldehyde-generating source. The shaped article formed from the deodorizing resin composition is not particularly limited to a specific form, and may be, for example, in the form of a particulate (e.g., a pellet), a fiber, a film (or a sheet), a bag or a container (a packing material), and others. Among the shaped articles, the particulate may be prepared by, for example, kneading and extruding components constituting the resin composition by an extruder, and crushing the pelletized or molten mixture. The fiber may be obtained by discharging a dope of the resin composition or a molten mixture thereof from a nozzle, and if necessary drawing the resulting matter.

In the embodiment (ii), the deodorizing property can be imparted to the substrate by coating or applying the deodorizing agent-containing solution on the surface of the substrate, impregnating the solution with the substrate, or laminating the deodorizing layer on the substrate. The resulting deodorizing substrate, if necessary, may be shaped, and may be located in the neighborhood of the aldehyde-generating source. The substrate may include, for example, a paper or a paper product (e.g., a wall paper), a fiber or a fiber product (e.g., a nonwoven fabric, and a filter made of a nonwoven fabric), a shaped article formed from a synthetic resin (e.g., a film, and a sheet), a wood building material and a product thereof (e.g., a building material such as a decorative laminate, a blockboard or a particle board), a metal (e.g., a metal board, and a metal foil), and a ceramics. The substrate may be porous.

In the method (1), the aldehyde odor may be efficiently eliminated by coating the aldehyde-generating source itself with the deodorizing agent, or by applying the deodorizing agent to a packaging or packing material (e.g., a wrapping paper, a wrapping bag, and a container) to package or pack the aldehyde-generating source. For example, even in the case of putting (or wrapping) a thermoplastic resin (e.g., a polyacetal resin pellet) in a high temperature state into the packing material, the packing material can efficiently trap an aldehyde to prevent a void due to packing from being filled with the aldehyde. Moreover, since the deodorizing agent can also coexist together with the generating source by locating a shaped article of a deodorizing resin composition such a pellet (a deodorizing particulate) close to an aldehyde-generating resin (e.g., a resin-shaped article, and a resin pellet), the deodorizing agent can efficiently eliminate the aldehyde odor and effectively inhibit spreading of the aldehyde. In particular, in the case of using, as the aldehyde-generating resin pellet, a resin pellet as the same series as the deodorizing particulate, the resin pellet may be also subjected to shaping (or molding) directly without fractionation or removal of the deodorizing particulate.

The method (2) of allowing the deodorizing agent to contain the aldehyde-generating source is effective in the case where the aldehyde-generating source is mainly an aldehyde-generating resin, and can inhibit the aldehyde generation from the resin or the resin-shaped article. In the method (2), the deodorizing agent (the deodorizing agent composition or the deodorizing resin composition) may be mixed with the aldehyde-generating resin to give a resin composition, and the resulting resin composition may be further shaped into a shaped article or the like. If necessary, the resulting resin composition or shaped article may have access to (or approach) the aldehyde-generating source (e.g., by coating, covering, and coexisting).

In the method (2), a resin composition in which aldehyde emission itself is remarkably inhibited can be obtained by mixing the deodorizing agent (the deodorizing agent composition or the deodorizing resin composition) with the aldehyde-generating resin. Further, in the case of using this resin composition, a variety of shaped articles in which aldehyde emission is inhibited can be produced as usage.

Moreover, the shaped article of formed from the polyacetal resin composition has excellent stability in an extrusion and/or molding process with having extremely small amount of emission (or generation) of formaldehyde. In other words, shaped articles molded from the conventional polyacetal resins containing antioxidants and other stabilizers liberate relatively large amounts of formaldehyde, cause corrosion and discoloration, and contaminate the living and working environment. For example, the formaldehyde emission from commercial ordinary polyacetal resin articles is about 2 to 5 µg per one cm² of surface area under dry conditions (in a constant-temperature dry atmosphere) and/or about 3 to 6 µg per one cm² of surface area under humid conditions (in a constant-temperature moisture-laden atmosphere).

On the other hand, since the polyacetal resin-shaped article of the present invention contains the specific hydrazine compound, the amount of formaldehyde emission from the shaped article can be effectively reduced. More specifically, under dry conditions, it is possible to achieve the formaldehyde emission amount of not more than 1. 5 µg (e.g., about 0 to 1.5 µg), preferably not more than 1.0 µg (e.g., about 0 to 1.0 µg), more preferably about 0 to 0.6 µg, usually about 0.001 to 1.0 µg, and further about 0 to 0.1 µg, per one cm² of surface area of the shaped article. Moreover, under humid conditions, the formaldehyde emission amount is not more than 2.5 µg (about 0 to 2 µg), preferably about 0 to 2 µg (e.g., about 0 to 1.2 µg), and more preferably about 0 to 0.4 µg, per one cm² of surface area of the shaped article. In the present invention, it is also possible to achieve the amount of formaldehyde emission in humid conditions of about 0 to 0.2 µg, and the emission amount may be usually not more than 1.2 µg (e.g., about 0 to 1.2 µg), and particularly about 0.001 to 1.2 µg.

The shaped article of the present invention should show the above-mentioned formaldehyde emission level under either dry conditions or humid conditions. In particular, the shaped article often shows the above formaldehyde emission level under both dry and humid conditions. The shaped article of the present invention is also preferably used as a material which can be adopted to more severe environment.

The formaldehyde emission under dry conditions can be determined as follows.

After the shaped article of polyacetal resin is cut if necessary and its surface area is measured, a suitable portion of the article (e.g. the amount equivalent to a surface area of about 10 to 50 cm²) is placed in a vessel (20 ml capacity) to seal and stand (or maintained) at a temperature of 80°C for 24 hours. Then, this sealed vessel is charged with 5 ml of water and the formaldehyde in the aqueous solution is assayed in accordance with JIS K0102, 29 (under the heading of Formaldehyde) to calculate the formaldehyde emission per unit surface area of the shaped article (µg/cm²).

The formaldehyde emission under humid conditions can be determined as follows.

After the shaped article of a polyacetal resin is cut if necessary and its surface area is measured, a suitable portion of the shaped article (e.g. the amount equivalent to a surface area of about 10 to 100 cm²) is suspended from the lid of a sealable vessel (1 L capacity) containing 50 ml of distilled water. After seal of the vessel, the vessel is allowed to stand (or maintained) in a constant temperature oven at 60°C for 3 hours. Thereafter, the vessel is allowed to stand at room temperature for 1 hour and the formaldehyde in the aqueous solution within the vessel is assayed in accordance with JIS K0102, 29 (under the heading of Formaldehyde) to calculate the formaldehyde emission per unit surface area of the article (µg/cm²).

The above quantitative limitation on formaldehyde emission in the present invention is valid as for the polyacetal resin and the specific hydrazine compound are contained not only for shaped articles available from polyacetal resin compositions comprising the conventional additives (conventional stabilizer, releasing agent, etc.), but also for shaped articles molded from comparable resin compositions containing an inorganic filler and/or other polymers, even if only a major part of the surface of the article (for example, 50 to 100% of the total surface area) is constituted by the polyacetal resin (for example, a multi-colored article or a coated article).

The deodorizing agent of the present invention comprises the specific hydrazine compound, and therefore can efficiently inhibit an aldehyde odor and can ensure a high heat resistance (a stability for heat fading). Moreover, even in the case of mixing the deodorizing agent or the deodorizing agent composition with the resin, the agent or the composition can certainly trap an aldehyde without deterioration of the resin properties. Further, the deodorizing agent or the deodorizing agent composition ensures to inhibit bleed out or volatilization and exhalation and to trap an aldehyde for reducing smells of the aldehyde over a long period of time. Furthermore, the deodorizing agent or the deodorizing agent composition can also significantly inhibit an aldehyde generation (or emission) from an aldehyde-generating resin by mixing with the resin.

### INDUSTRIAL APPLICABILITY

The deodorizing agent (or the deodorizing agent composition) and the deodorizing composition of the present invention is useful for application used close on the aldehyde-generating source, for example, a packaging material or a packing material (e.g., a paper, a bag, and a container), a building material (e.g., a wall paper, and a blockboard), a filter (e.g., a filter made from a nonwoven fabric), or a particulate shaped article such as a resin pellet.

The resin composition (in particular, the polyacetal resin composition) of the present invention is useful in fabricating a variety of shaped articles by a conventional molding method (e.g., injection molding, extrusion molding, compression molding, blow molding, vacuum molding, foam molding, rotation molding, and gas injection molding).

Moreover, the shaped article of the polyacetal resin of the present invention is available for application in any field of use where formaldehyde is objectionable (for example, knobs and levers for use as bicycle parts) and can also be used advantageously as parts and members in a variety of fields inclusive of automotive parts (e.g., car interior parts such as inner handles, fuel trunk openers, various switches and levers; electrical system parts such as meters and connectors; in-vehicle electrical and electronic parts related to audio equipment and car navigation equipment, parts in contact with metals represented by the window regulator carrier plate, and mechanical parts such as door lock actuator parts, mirror parts, wiper motor system parts, and fuel system parts), electrical and/or electronic component parts [for example, driving component parts and driven component parts, e.g., parts or members fitted with a number of metal contacts (e.g., audio equipment, video equipment such as video cameras, office automation (OA) equipment such as copying machines, facsimiles and computers, toys actuated by the driving force of an electric motor or a spring, telephones, keyboards as an accessory to a computer or the like, more specifically, a chassis, a gear, a lever, a cam, a pulley, and a bearing), building materials and pipeline installation parts (e.g., lighting equipment parts, interior architectural members (such as fittings, fixtures and furnishings), piping, cock, faucet, and rest room (lavatory)-related parts), products related to daily living, cosmetic products, and medical (health care and/or therapy) devices (for example, fasteners, stationery, chapstick or lipstick cases, cleansing device, water purifiers (or water cleaners), spray nozzles, spray devices or containers, aerosol containers, general vessels, and syringe holders), food grade parts [for example, devices for eating (e.g., containers, trays, spoons), utensils (e.g., air rollers, mesh rollers, and cleavers or kitchen knives), cocks (e.g., cocks for drinking water), packages, cooking appliance parts (e.g., parts for an ice-cream making machine), freezer parts, washing machine parts (e.g., vegetable washers, dish washers, and bottle washers), drying machine parts (e.g. , dish drying machines), water purifier parts, pot parts, thermos bottle parts, jar parts, and mixer parts], and photographic parts (e.g., camera parts, and photo film parts). Moreover, the shaped article is applicable for optical and magnetic media parts (e.g., metal thin-film magnetic tape cassettes, magnetic disk cartridges, and opticomagnetic disc cartridges), for example, metal tape cassettes for music, digital audio tape cassettes, 8 mm video tape cassettes, floppy (registered trademark) disk cartridges, and minidisk cartridges.

### EXAMPLES

The following examples are intended to describe this invention in further detail and should by no means be interpreted as defining the scope of the invention.

Incidentally, referring to the examples and comparative examples, the moldability (the amount of the deposit on the mold), the amount of emission of formaldehyde from the molded (or shaped) articles under dry and humid conditions, and the bleeding property were evaluated based on the following methods.

### [Moldability (the amount of the deposit on the mold)]

A pellet formed with a polyacetal resin composition was continuously or successively shaped or molded by using a 30 t injection molding machine (100 shots) to obtain a certain-shaped article (20 mm in diameter and 1 mm in thickness), and the degree of the deposition on the mold was evaluated and classified into five grades. Incidentally, the smaller the number of the levels is, the lower or smaller the amount of the deposit (i.e., mold deposit) is.

### [Amount of formaldehyde emission from shaped article in dry conditions]

Each resin sample consisting of 10 test pieces (one test piece: 2 mm x 2 mm x 50 mm; total surface area: about 40 cm²) was placed in a vessel (capacity 20 ml) to seal and heated in a constant temperature oven at 80°C for 24 hours. After air-cooling to room temperature, 5 ml of distilled water was injected into the vessel using a syringe. The formaldehyde content of this aqueous solution was determined in accordance with JIS K0102, 29 (under the heading of Formaldehyde) and the formaldehyde gas emission per surface area (µg/cm²) was calculated.

### [Amount of formaldehyde emission from shaped article in humid conditions, and the bleeding property]

Two plate test pieces (one piece: 100 mm x 40 mm x 2 mm; total surface area of 85.6 cm²) were suspended from a lid of a polyethylene bottle (capacity 1 L) containing 50 ml of distilled water. The bottle was sealed to stand in a constant temperature oven at 60°C for 3 hours, followed by standing for 1 hour at room temperature. The formaldehyde content of the aqueous solution in the bottle was determined in accordance with JIS K0102, 29 (under the heading of Formaldehyde) and the formaldehyde gas emission per surface area of the article (µg/cm²) was calculated.

Further, the surface of the plate test piece (shaped article) after the test was visually observed, and the degree of the bleeding was evaluated based on the following criteria.
"A": No bleeding was observed.
"B": Slight bleeding was observed.
"C": Extremely heavy bleeding was observed.

### Examples 1 to 10 and Comparative Examples 1 to 2

To 100 parts by weight of a polyacetal resin copolymer was preblended (or premixed) a hydrazine compound in the proportions indicated in Table 1. Concerning each of thus obtained mixtures, the mixture was supplied through a main feed port of a biaxial extruder (30 mm diameter) having one vent port, and melt-mixed (extrusion condition: L/D=35, extrusion temperature: 200°C, screw rotation frequency: 100 rpm, vent vacuum: 20 mmHg (2.66 kPa), discharging rate: 15 kg/hr, and average residence time: 100 seconds) to prepare a pelletized composition.

From thus obtained pellet compositions, prescribed test pieces were fabricated with an injection molding machine, and concerning each test piece, the moldability (mold deposit), the amount of formaldehyde emission from the test piece, and the bleeding property were measured.

For comparison, these characteristics were evaluated in the same manner as the above-mentioned operation except for using the polyacetal resin copolymer alone having no the hydrazine compound (Comparative Examples 1 and 2). Incidentally, in each of these Comparative Examples, the resin was decomposed in both steps of preparing a pellet composition and molding a test piece, resulted in foaming, and therefore, the uniform shaped articles could not be obtained from the resin.

The results are shown in Table 1.

**Table 1**

| | Examples | | | | | | | | | | Comparative Examples | |
|---|---|---|---|---|---|---|---|---|---|---|---|---|
| | 1 | 2 | 3 | 4 | 5 | 6 | 7 | 8 | 9 | 10 | 1 | 2 |
| Polyacetal resin "a" (parts by weight) | a-1 | a-1 | a-1 | a-1 | a-1 | a-1 | a-1 | a-1 | a-1 | a-2 | a-1 | a-2 |
| | 100 | 100 | 100 | 100 | 100 | 100 | 100 | 100 | 100 | 100 | 100 | 100 |
| Hydrazine compound "b" (parts by weight) | b-1 | b-1 | b-2 | b-3 | b-4 | b-5 | b-6 | b-7 | b-8 | b-1 | - | - |
| | 0.2 | 0.5 | 0.5 | 0.5 | 0.5 | 0.5 | 0.5 | 0.5 | 0.5 | 0.5 | | |
| Moldability (Mold deposit) | 3 | 2 | 2 | 2 | 2 | 2 | 2 | 2 | 2 | 2 | 2 | 2 |
| Amount of formaldehyde emission Dry (µg/cm²) | 0.20 | 0.03 | 0.03 | 0.04 | 0.06 | 0.04 | 0.04 | 0.05 | 0.03 | 0.03 | 7.81 | 6.87 |
| Amount of formaldehyde emission Humid (µg/cm²) | 0.35 | 0.05 | 0.06 | 0.06 | 0.08 | 0.06 | 0.07 | 0.07 | 0.05 | 0.04 | 5.55 | 5.02 |
| Bleeding property | A | B | B | B | B | B | B | B | B | B | A | A |

As apparent from the Table and foaming from the resin in Comparative Examples, the compositions of Examples using the specific hydrazine compound can reduce the formaldehyde emission to extremely small amount as compared with those of Comparative Examples, and can significantly improve the working and using environments. Thus, the specific hydrazine compound has an extremely remarkably inhibitory effect on the formaldehyde emission.

Moreover, the mold deposit in each of Examples was as small as that in each of Comparative Examples having no additive(s). Furthermore, since no bleeding was observed (Example 1) or slight bleeding was observed (Examples 2 to 10), the compositions of Examples ensured improvement in the quality of the shaped articles.

Incidentally, the composition obtained in the same manner as Example 1 except that the proportion of the hydrazine compound was 0.01 part by weight, 1 part by weight or 5 parts by weight showed the results similar to Example 1. Moreover, the composition produced in the same manner as Example 1 except that 0.1 part by weight of sodium acetate was used in combination with the hydrazine compound obtained the same results as Example 1.

### Examples 11 to 34 and Comparative Examples 3 to 14

To 100 parts by weight of a polyacetal resin copolymerwerepreblended (or premixed) a specific hydrazine compound, an antioxidant, a processing stabilizer and a heat stabilizer in the proportions indicated in Tables 2 and 3. Concerning each of thus obtained mixtures, the mixture was supplied through a main feed port of a biaxial extruder (30 mm diameter) having one vent port, and melt-mixed (extrusion condition: L/D=35, extrusion temperature: 200°C, screw rotation frequency: 100 rpm, vent vacuum: 20 mmHg (2.66 kPa), discharging rate: 15 kg/hr, and average residence time: 100 seconds) to prepare a pelletized composition. From thus obtained pellet compositions, prescribed test pieces were fabricated with an injection molding machine, and concerning each test piece, the moldability (mold deposit), the amount of formaldehyde emission from the test piece, and the bleeding property were evaluated. The results are shown in Tables 2 and 3.

For comparison, samples prepared without addition of the specific hydrazine compound (Comparative Examples 3 to 10), and samples prepared with addition of other hydrazine compound instead of the specific hydrazine compound (Comparative Examples 11 to 14) were evaluated in the same manner as Examples mentioned above. The results are shown in Table 3.

### Examples 35 and 36

For preparing the same compositions as Examples 33 and 34, to 95 parts by weight of the polyacetal resin copolymer were preblended an antioxidant, a processing stabilizer and a heat stabilizer. Then, concerning each of thus obtained mixtures, the mixture was supplied through a main feed port of a biaxial extruder (30 mm diameter) having one vent port, and a mixture of 5 parts by weight of the polyacetal resin copolymer in a particulate form and the specific hydrazine compound was supplied through a side feed port located in the downstream of the vent port. The resulting matter was melt-mixed in the following conditions (extrusion condition: L/D=35, extrusion temperature: 200°C, screw rotation frequency: 100 rpm, vent vacuum: 20 mmHg (2.66 kPa), discharging rate: 15 kg/hr, and average residence time: 100 seconds) to prepare a pelletized composition.

From thus obtained pellet compositions, prescribed test pieces were fabricated with an injection molding machine, and concerning each test piece, the moldability (mold deposit), the amount of formaldehyde emission from the test piece, and the bleeding property were measured. The results are shown in Table 3.

**Table 2**

| | | | | | | | | | | Examples | | | | | | | | | | |
|---|---|---|---|---|---|---|---|---|---|---|---|---|---|---|---|---|---|---|---|---|
| | 11 | 12 | 13 | 14 | 15 | 16 | 17 | 18 | 19 | 20 | 21 | 22 | 23 | 24 | 25 | 26 | 27 | 28 | 29 | 30 |
| Polyacetal resin "a" (parts by weight) | a-1 | a-1 | a-1 | a-1 | a-1 | a-1 | a-1 | a-1 | a-1 | a-1 | a-1 | a-2 | a-1 | a-1 | a-1 | a-1 | a-1 | a-1 | a-1 | a-1 |
| | 100 | 100 | 100 | 100 | 100 | 100 | 100 | 100 | 100 | 100 | 100 | 100 | 100 | 100 | 100 | 100 | 100 | 100 | 100 | 100 |
| Hydrazine compound "b" (parts by weight) | b-1 | b-1 | b-1 | b-1 | b-2 | b-3 | b-4 | b-5 | b-6 | b-7 | b-8 | b-1 | b-1 | b-1 | b-1 | b-1 | b-1 | b-1 | b-1 | b-1 |
| | 0.2 | 0.2 | 0.2 | 0.2 | 0.2 | 0.2 | 0.2 | 0.2 | 0.2 | 0.2 | 0.2 | 0.2 | 0.2 | 0.2 | 0.2 | 0.2 | 0.3 | 0.3 | 0.3 | 0.3 |
| Antioxidant "c" (parts by weight) | c-1 | c-1 | c-1 | c-1 | c-1 | c-1 | c-1 | c-1 | c-1 | c-1 | c-1 | c-1 | c-2 | c-1 | c-1 | c-1 | c-1 | c-1 | c-1 | c-1 |
| | 0.3 | 0.3 | 0.3 | 0.3 | 0.3 | 0.3 | 0.3 | 0.3 | 0.3 | 0.3 | 0.3 | 0.3 | 0.3 | 0.3 | 0.3 | 0.3 | 0.3 | 0.3 | 0.3 | 0.3 |
| Processing stabilizer "d" (parts by weight) | - | d-1 | - | d-1 | d-1 | d-1 | d-1 | d-1 | d-1 | d-1 | d-1 | d-1 | d-1 | d-2 | d-3 | d-4 | d-1 | d-1 | d-2 | d-2 0 |
| | | 0.2 | | 0.2 | 0.2 | 0.2 | 0.2 | 0.2 | 0.2 | 0.2 | 0.2 | 0.2 | 0.2 | 0.2 | 0.2 | 0.2 | 0.2 | 0.2 | 0.2 | .2 |
| Heat stabilizer "e" (parts by weight) | - | - | e-1 | e-1 | e-1 | e-1 | e-1 | e-1 | e-1 | e-1 | e-1 | e-1 | e-1 | e-1 | e-1 | e-1 | e-1 | e-2 | e-3 | e-4 |
| | | | 0.1 | 0.1 | 0.1 | 0.1 | 0.1 | 0.1 | 0.1 | 0.1 | 0.1 | 0.1 | 0.1 | 0.1 | 0.1 | 0.1 | 0.03 | 0.03 | 0.03 | 0.03 |
| Moldability (Mold deposit) | 1 | 1 | 1 | 1 | 1 | 1 | 1 | 1 | 1 | 2 | 1 | 1 | 1 | 1 | 1 | 1 | 2 | 1 | 1 | 2 |
| Amount of formaldehyde emission Dry (µg/cm²) | 0.07 | 0.05 | 0.04 | 0.03 | 0.03 | 0.04 | 0.05 | 0.03 | 0.03 | 0.04 | 0.03 | 0.03 | 0.06 | 0.04 | 0.03 | 0.04 | 0.02 | 0.04 | 0.05 | 0.04 |
| Amount of formaldehyde emission Humid (µg/cm²) | | | | | | | | | | | | | | | | | | | | |
| | 0.12 | 0.09 | 0.08 | 0.07 | 0.07 | 0.07 | 0.09 | 0.07 | 0.08 | 0.07 | 0.06 | 0.06 | 0.08 | 0.07 | 0.06 | 0.05 | 0.04 | 0.07 | 0.08 | 0.08 |
| | | | | | | | | | | | | | | | | | | | | |
| Bleeding property | B | A | B | A | A | A | A | A | A | A | A | A | A | A | A | A | A | A | A | A |

**Table 3**

| | Examples | | | | | | Comparative Examples | | | | | | | | | | | |
|---|---|---|---|---|---|---|---|---|---|---|---|---|---|---|---|---|---|---|
| | 31 | 32 | 33 | 34 | 35 | 36 | 3 | 4 | 5 | 6 | 7 | 8 | 9 | 10 | 11 | 12 | 13 | 14 |
| Polyacetal resin "a" (parts by weight) | a-1 | a-1 | a-1 | a-1 | a-1 | a-1 | a-1 | a-1 | a-1 | a-1 | a-2 | a-1 | a-1 | a-1 | a-1 | a-1 | a-1 | a-1 |
| | 100 | 100 | 100 | 100 | 100 | 100 | 100 | 100 | 100 | 100 | 100 | 100 | 100 | 100 | 100 | 100 | 100 | 100 |
| Hydrazine compound "b" (parts by weight) | b-1 | b-5 | b-1 | b-8 | b-1 | b-8 | - | - | - | - | - | - | - | - | b-9 | b-9 | b-10 | b-11 |
| | 0.1 | 0.1 | 0.1 | 0.2 | 0.2 | 0.2 | | | | | | | | | 0.2 | 0.2 | 0.2 | 0.2 |
| Antioxidant "c" (parts by weight) | c-1 | c-1 | c-1 | c-1 | c-1 | c-1 | c-1 | c-1 | c-1 | c-1 | c-1 | c-2 | c-1 | c-1 | c-1 | c-1 | c-1 | c-1 |
| | 0.3 | 0.3 | 0.3 | 0.3 | 0.3 | 0.3 | 0.3 | 0.3 | 0.3 | 0.3 | 0.3 | 0.3 | 0.3 | 0.3 | 0.3 | 0.3 | 0.3 | 0.3 |
| Processing stabilizer "d" (parts by weight) | d-1 | d-2 | d-1 | d-2 | d-1 | d-2 | - | d-1 | - | d-1 | d-1 | d-1 | d-2 | d-1 | d-2 | d-1 | d-1 | d-1 |
| | 0.2 | 0.2 | 0.2 | 0.2 | 0.2 | 0.2 | | 0.2 | | 0.2 | 0.2 | 0.2 | 0.2 | 0.2 | 0.2 | 0.2 | 0.2 | 0.2 |
| Heat stabilizer "e" (parts by weight) | e-1 e-6 | e-1 e-7 | e-1 | e-5 | e-1 | e-5 | - | - | e-1 0 | e-1 | e-1 | e-1 | e-1 | e-2 | e-2 | e-1 | e-2 | e-1 |
| | 0.1 0.05 | 0.1 0.05 | 0.1 | 0.03 | 0.1 | 0.03 | | | .1 | 0.1 | 0.1 | 0.1 | 0.1 | 0.1 | 0.1 | 0.1 | 0.1 | 0.1 |
| Moldability (Mold deposit) | 1 | 1 | 2 | 1 | 1 | 1 | 2 | 2 | 2 | 1 | 1 | 1 | 1 | 1 | 1 | 1 | 1 | 5 |
| Amount of formaldehyde emission Dry (µg/cm²) | 0.04 | 0.05 | 0.07 | 0.08 | 0.04 | 0.04 | 3.97 | 3.65 | 3.82 | 3.70 | 3.51 | 3.68 | 3.81 | 3.98 | 3.68 | 3.73 | 5.32 | 0.04 |
| Amount of formaldehyde emission Humid (µg/cm²) | 0.07 | 0.06 | 0.10 | 0.09 | 0.08 | 0.07 | 1.54 | 1.33 | 1.43 | 1.35 | 1.28 | 1.31 | 1.45 | 1.47 | 1.37 | 1.38 | 2.90 | 0.08 |
| Bleeding property | A | A | A | A | A | A | A | A | A | A | A | A | A | A | A | A | A | C |

As apparent from the Tables, each of the compositions of Examples could reduce the formaldehyde emission to extremely small amount as compared with those of Comparative Examples (Comparative Examples 3 to 13), and can significantly improve the working and using environments. In Comparative Example 14, using adipic acid hydrazide, the antioxidant, the processing stabilizer and the heat stabilizer in combination, the amount of the formaldehyde emission was low, while the mold deposit and the bleeding remarkably occurred.

On the other hand, in each of Examples, the moldability (mold deposit) was improved and also the bleeding property was improved, as a result, the quality of the obtained shaped article was enhanced. In particular, according to Examples, the inhibitory effect on the formaldehyde emission was synergistically improved by using the specific hydrazine compound in combination with the antioxidant, the processing stabilizer and/or the heat stabilizer.

Incidentally, the composition obtained in the same manner as Example 11 except that the proportion of the antioxidant was 1 part by weight showed the results similar to Example 11. The composition produced in the same manner as Example 12 except that the proportion of the processing stabilizer was 0.01 part by weight, 1 part by weight or 3 parts by weight obtained the same results as Example 12. The composition obtained in the same manner as Example 13 except that the proportion of the heat stabilizer was 1 or 2 part by weight showed the results similar to Example 13.

### Examples 37 to 42 and Comparative Examples 15 to 20

Pellet compositions having proportions shown in Table 4 were prepared, and concerning each of the compositions, a test piece was molded and properties of the test piece were evaluated in the same manner as in Example 1.

Incidentally, in each of Examples 37 to 41 and Comparative Examples 15 to 19, a pellet composition was prepared in the same manner as Examples 11 to 34 by preblending all constitutive components and feeding the blended product through the main feed port. Moreover, in each of Example 42 and Comparative Example 20, a pellet composition was prepared in the same manner as Examples 35 and 36 except for preblending components other than a glass fiber (h-3) and supplying the blended mixture through a main feed port, and for feeding the glass fiber (h-3) through a side feed port in the upstream of an exhaust vent port. The results are shown in Table 4.

**Table 4**

| | Examples | | | | | | Comparative Examples | | | | | |
|---|---|---|---|---|---|---|---|---|---|---|---|---|
| | 37 | 38 | 39 | 40 | 41 | 42 | 15 | 16 | 17 | 18 | 19 | 20 |
| Polyacetal resin "a" (parts by weight) | a-1 | a-1 | a-1 | a-1 | a-1 | a-1 | a-1 | a-1 | a-1 | a-1 | a-1 | a-1 |
| | 100 | 100 | 100 | 100 | 100 | 100 | 100 | 100 | 100 | 100 | 100 | 100 |
| Hydrazine compound "b" (parts by weight) | b-1 | b-1 | b-8 | b-1 | b-1 | b-1 | - | - | - | - | - | - |
| | 0.3 | 0.3 | 0.3 | 0.3 | 0.3 | 0.3 | | | | | | |
| Antioxidant "c" (parts by weight) | c-1 | c-1 | c-1 | c-1 | c-1 | c-1 | c-1 | c-1 | c-1 | c-1 | c-1 0 | c-1 |
| | 0.3 | 0.03 | 0.03 | 0.03 | 0.2 | 0.2 | 0.3 | 0.03 | 0.03 | 0.03 | .3 | 0.3 |
| Processing stabilizer "d" (parts by weight) | d-1 | d-1 | d-1 | d-1 | d-1 | d-1 | d-1 | d-1 | d-1 | d-1 | d-1 | d-1 |
| | 0.2 | 0.2 | 0.2 | 0.2 | 0.2 | 0.2 | 0.2 | 0.2 | 0.2 | 0.2 | 0.2 | 0.2 |
| Heat stabilizer "e" (parts by weight) | e-1 e-8 | e-1 | e-1 | e-1 | e-1 | e-1 | e-1 e-8 | e-1 | e-1 | e-1 | e-1 | e-1 |
| | 0.1 0.05 | 0.1 | 0.1 | 0.1 | 0.1 | 0.1 | 0.1 0.05 | 0.1 | 0.1 | 0.1 | 0.1 | 0.1 |
| Coloring agent "f" (parts by weight) | f-1 | - | - | - | - | - | f-1 | - | - | - | - | - |
| | 0.5 | | | | | | 0.5 | | | | | |
| Weather (light)-resistant stabilizer "g" (parts by weight) | - | g-1 g-2 | g-1 g-3 | g-1 g-2 | - | - | - | g-1 g-2 | g-1 g-3 | g-1 g-2 | - | - |
| | | 0.4 0.2 | 0.4 0.2 | 0.4 0.2 | | | | 0.4 0.2 | 0.4 0.2 | 0.4 0.2 | | |
| Other additive "h" (parts by weight) | - | - | - | h-1 | h-2 | h-3 | - | - | - | h-1 | h-2 | h-3 |
| | | | | 5.0 | 15.0 | 33.0 | | | | 5.0 | 15.0 | 33.0 |
| Moldability (Mold deposit) | 1 | 1 | 1 | 1 | 1 | 1 | 2 | 2 | 2 | 3 | 2 | 3 |
| Amount of formaldehyde emission, Dry (µg/cm²) | 0.11 | 0.15 | 0.13 | 0.06 | 0.03 | 0.04 | 6.70 | 5.79 | 5.87 | 8.83 | 5.02 | 6.20 |
| Amount of formaldehyde emission, Humid (µg/cm²) | 0.14 | 0.19 | 0.17 | 0.09 | 0.04 | 0.06 | 4.13 | 3.10 | 3.33 | 5.14 | 3.11 | 4.32 |
| Bleeding property | A | A | A | A | A | A | A | A | A | A | A | A |

As apparent from the Table, each of the compositions of Examples could reduce the formaldehyde emission to extremely small amount as compared with those of Comparative Examples, and can significantly improve the working and using environments. Further, in each of Examples, the mold deposit was inhibited and moldability was improved. Furthermore, the bleeding property was inhibited, as a result, the quality of the obtained shaped article could be enhanced. Moreover, in each of Examples, the inhibitory effect on the formaldehyde emission was not deteriorated even in the case of blending the antioxidant, the processing stabilizer, the heat stabilizer, the coloring agent, the weather (light)-resistant stabilizer, and other additive(s) with the polyacetal resin and the hydrazine compound.

Incidentally, the compositions of Examples was not different from those of Comparative Examples in the coloring property due to addition of the coloring agent, the weather (light)-resistance stability due to addition of the weather (light)-resistant stabilizer, and various properties (e.g., impact resistance, and mechanical strength) due to addition of other additive(s). It is also apparent from such facts that the addition of the specific hydrazine compound did not have adverse affects on the properties of the composition (e.g., the coloring property, the weather (light)-resistance stability, and the impact resistance).

Incidentally, the composition obtained in the same manner as Example 38 except that 0.1 part by weight of (g-1) or 2 parts by weight of (g-1) and 1 part by weight of (g-2) were used instead of 0.4 part by weight of (g-1) and 0.2 part by weight of (g-2) in the weather (light)-resistant stabilizer showed the results similar to Example 38. The composition produced in the same manner as Example 37 except that the proportion of the coloring agent was 0.1 or 3 parts by weight obtained the same results as Example 37. The composition obtained in the same manner as Example 40 except that 0.5 part by weight of (h-1) or 20 parts by weight of (h-2) was used instead of 5.0 parts by weight of (h-1) in the impact resistance improver showed the results similar to Example 40. The composition produced in the same manner as Example 42 except that the proportion of the glass fiber (h-3) was 5 or 60 parts by weight obtained the same results as Example 42.

### Example 43

The pellet of the polyacetal resin composition obtained in Example 14 was supplied through a main feed port of a biaxial extruder (30 mm diameter) having one vent port, and further melt-mixed (extrusion condition: L/D=35, extrusion temperature: 200°C, screwrotation frequency: 100 rpm, vent vacuum: 20 mmHg (2.66 kPa), discharging rate: 15 kg/hr, and average residence time: 100 seconds) to give a pelletized composition prepared under the condition of a total residence time of 200 seconds in the extruder.

From thus obtained pellet composition, a prescribed test piece was fabricated in the same manner as Example 14 to perform the evaluation of properties. The amount of the formaldehyde emission from the test piece was 0.08 µg/cm² in the dry condition and 0.19 µg/cm² in the humid condition. The composition expressed no bleeding (bleeding property: "A"), and had a high moldability (a small mold deposit) (evaluation criteria of moldability: "1").

### Example 44

One hundred (100) parts by weight of the pellet of the polyacetal resin composition obtained in Comparative Example 6 (the pellet having no the hydrazine compound) and 0.1 part by weight of the hydrazine compound (b-1) were put in a polyethylene bag and blended to prepare a blended product. A test piece was fabricated in the same manner as Comparative Example 6 except for using he resulting blended product (resin composition), and was evaluated for the properties. The formaldehyde emission from the test piece was 0.03 µg/cm² in the dry condition and 0.08 µg/cm² in the humid condition, and the bleeding property was evaluated as "A". Moreover, the moldability (mold deposit) was determined as "1" in the evaluation criteria of moldability.

### Example 45

An operation was carried out in the same manner as Example 44 except for using 0.1 part by weight of the hydrazine compound (b-8) instead of the hydrazine compound (b-1), and the properties were evaluated. The formaldehyde emission from the test piece was 0.02 µg/cm² in the dry condition and 0.07 µg/cm² in the humid condition, and the bleeding property was evaluated as "A". Moreover, the moldability (mold deposit) was determined as "1" in the evaluation criteria of moldability.

### Examples 46 to 47 and Comparative Example 21

Two (2) grams of the hydrazine compound (b-1) (Example 46) or 2 g of the hydrazine compound (b-8) (Example 47) was charged in an open petri dish, and held on a center slate of a glass desiccator (6L) equipped with an upper opening and a side opening. Then, 20 ppm of formaldehyde gas was filled in the desiccator, and the desiccator was sealed. Under this state the desiccator was allowed to stand at 30°C for 3 hours, and then the concentration of residual formaldehyde in the desiccator was measured by a gas detecting tube. As a result, the residual formaldehyde concentration in the both Examples 46 and 47 is not more than 0.05 ppm.

For comparison, the residual formaldehyde concentration was measured in the same manner as the above operation without using the hydrazine compound, and the residual formaldehyde concentration was determined as 19 ppm (Comparative Example 21).

Thus, it becomes clear that the specific hydrazine compound has odor-eliminating and removing effects on formaldehyde under coexistence with formaldehyde.

### Example 48 and Comparative Example 22

To a mixture of 1 g of the hydrazine compound (b-8) as a deodorizing agent and 0.1 g of sodium acetate as a deodorizing auxiliary was added water to prepare a aqueous solution in which the concentration of the hydrazine compound was about 10% by weight. A nonwoven fabric made from a polyester (10 cm x 10 cm) was impregnated with the aqueous solution, and dried.

The obtained deodorizing agent-impregnating nonwoven fabric was held on a center slate of a glass desiccator (6L) equipped with an upper opening and a side opening. Then, 20 ppm of formaldehyde gas was filled in the desiccator, and the desiccator was sealed. Under this state the desiccator was allowed to stand at 30°C for 3 hours, and then the concentration of residual formaldehyde in the desiccator was measured by a gas detecting tube. The residual formaldehyde concentration was not more than 0.05 ppm, and therefore, it becomes clear that the hydrazine compound and sodium acetate as a deodorizing auxiliary have odor-eliminating and removing effects on formaldehyde even under the state held on the nonwoven fabric.

Incidentally, the same operation as mentioned above except that the proportion of sodium acetate was 0.01 g, 0. 5 g or 1 g relative to 1 g of the hydrazine compound brought the same results as mentioned above.

Moreover, an operation in the same manner as mentioned above except that 0.1 g of magnesium stearate, benzoguanamine, magnesium oxide, glycerin or resorcin was used instead of 0.1 g of sodium acetate brought the results similar to the above-mentioned results. An operation in the same manner as mentioned above except that 1 g of cyclodextrin was used instead of 0.1 g of sodium acetate brought the results similar to the above-mentioned results.

For comparison, the residual formaldehyde concentration was measured in the same manner as the above operation except for using a polyester nonwoven fabric not impregnated with the aqueous solution, and the residual formaldehyde concentration was determined as 18 ppm.

Incidentally, the polyacetal resin copolymers, the hydrazine compounds, the antioxidants, the processing stabilizers, the heat stabilizers, the coloring agents, the weather (light)-resistant stabilizers, and other additives used in Examples and Comparative Examples were shown as follows.
1. Polyacetal copolymer "a"
   (a-1) : Polyacetal resin copolymer (melt index = 9 g/10 min.)
   (a-2): Polyacetal resin copolymer (melt index = 27 g/10 min.)

   Incidentally, the melt index is a value (g/10 min.) determined according to ASTM-D1238, and in the condition of using 2169 g of the resin at a temperature of 190°C.
2. Hydrazine compound "b"
   [Specific hydrazine compound]
   (b-1): β-(3,5-Di-t-butyl-4-hydroxyphenyl)propionyl hydrazine
   (b-2): β-(3-Methyl-5-t-butyl-4-hydroxyphenyl)propionyl hydrazine
   (b-3): β-Methyl-β-(3-t-butyl-4-hydroxyphenyl)propionyl hydrazine
   (b-4): N-Monosalicyloyldodecane diacid dihydrazide
   (b-5): N-Mono[β-(3,5-di-t-butyl-4-hydroxyphenyl)propionyl]sebacic acid dihydrazide
   (b-6): N-Mono[β-(3-methyl-5-t-butyl-4-hydroxyphenyl)propionyl]dodecane diacid dihydrazide
   (b-7): Oligoadipic acid dihydrazide
      H₂NHNOC-(CH₂)₄-CO(-NHNHOC-(CH₂)₄-CO)ᵣ-NHNH-(CH₂)₄-CONHNH₂ prepared from 2 mol of adipic acid dihydrazide and 1 mol of adipic acid dichloride (in the formula, "r" is 1)
   (b-8): 4-Aminourazole
      [Other hydrazine compound]
   (b-9): N,N'-Bis[β-(3,5-di-t-butyl-4-hydroxyphenyl)propionyl]hydrazine [manufactured by Ciba Specialty Chemicals K.K., "Irganox MD1024"]
   (b-10): N,N'-Bis(salicyloyl)dodecane diacid dihydrazide [manufactured by Asahi Denka Kogyo K.K. , "ADKSTAB CDA-6"]
   (b-11): Adipic acid dihydrazide
3. Antioxidant "c"
   (c-1): Triethylene glycol bis[3-(3-t-butyl-5-methyl-4-hydroxyphenyl)propionate]
   (c-2): Pentaerythritol tetrakis[3-(3,5-di-t-butyl-4-hydroxyphenyl)propionate]
4. Processing stabilizer "d"
   (d-1): Ethylene bis-stearyl amide
   (d-2): Glycerin monostearate
   (d-3): Montanic ester [manufactured by Toyo-Petrolite Co., Ltd., "LUZA WAX-EP"]
   (d-4): Polyethylene oxide [molecular weight: 35000]
5. Heat stabilizer (metal salt of organic carboxylic acid, alkaline earth metal salt, basic nitrogen compound) "e"
   (e-1): Calcium citrate
   (e-2): Calcium 12-hydroxystearate
   (e-3): Magnesium stearate
   (e-4): Calcium stearate
   (e-5): Magnesium oxide
   (e-6): Biurea
   (e-7): Polyamide 6-66-610
   (e-8): Polyamide 66 (average particle size: 3 µm)
6. Coloring agent "f"
   (f-1): Carbon black (acetylene black)
7. Weather (light)-resistant stabilizer "g"
   (g-1): 2-[2'-Hydroxy-3',5'-bis(a,a-dimethylbenzyl)phenyl]benzotriazole
   (g-2): Bis(1,2,2,6,6-pentamethyl-4-piperidyl)sebacate
   (g-3): Bis(2,2,6,6-tetramethyl-4-piperidyl)sebacate
8. Other additive "h"
   (h-1): Acrylic core-shell polymer [manufactured by Takeda Chemical Industries, Ltd., "STAPHYLOID PO"]
   (h-2): Thermoplastic polyurethane [manufactured by Nippon Miractran Co., Ltd., "MIRACTRAN E"]
   (h-3): Glass fiber [chopped strand having 9 µm in diameter and 3 mm long]

## Claims

1. An aldehyde odor-deodorizing agent which comprises at least one hydrazine compound selected from the group consisting of a polycarboxylic acid hydrazide having an aryl group, a hydrazide of an arylalkanecarboxylic acid (A1), a non-acrylic polyhydrazide (A2), and an aminourazole compound (A3),
wherein the polycarboxylic acid hydrazide having an aryl group and the hydrazide of the arylalkanecarboxylic acid (A1) have at least one hydroxyl group or a group derived from the hydroxy group on the aryl group(s),
the polycarboxylic acid hydrazide having an aryl group and the hydrazide of the arylalkanecarboxylic acid (A1) have at least one free hydrazino group, and
the non-acrylic polyhydrazide (A2) comprises a polyhydrazide shown by the following formula (2): 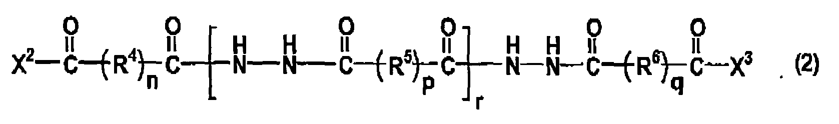 wherein R⁴ to R⁶ are the same or different and each represents a divalent hydrocarbon group or heterocyclic group"n", "p" and "q" are the same or different and each denotes 0 or 1, "r" denotes an integer of not smaller than 0;
X² and X³ are the same or different and each represents a halogen atom, an amino group, a hydrazino group, or a group -OX⁴ wherein X⁴ represents a hydrogen atom, a hydrocarbon group, an alkali metal atom, or an alkaline earth metal atom, and with the proviso that at least one of X² and X³ is a hydrazino group.

2. A deodorizing agent according to claim 1, wherein the polycarboxylic acid hydrazide having an aryl group and the hydrazide of the arylalkanecarboxylic acid (A1) have at least one group selected from a hydroxyl group, an alkoxy group, and an acyloxy group on the aryl group(s).

3. A deodorizing agent according to claim 1, wherein the polycarboxylic acid hydrazide having an aryl group and the hydrazide of the arylalkanecarboxylic acid (A1) comprises a carboxylic acid hydrazide shown by the following formula (1): 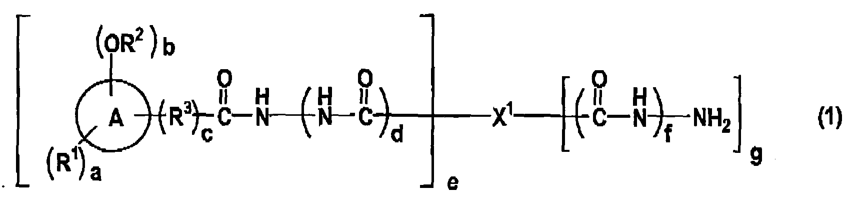 wherein R¹ represents a hydrocarbon group, R² represents a hydrogen atom, an alkyl group or an acyl group, R³ represents an alkylene group, a ring A represents an aromatic ring, X¹ represents a direct bond or a polyvalent organic group; "a" denotes an integer of 0 to 6, "b" denotes an integer of 1 to 4; "c" denotes 0 or 1, "d" is 1 when "c" is 0, "d" is 0 or 1 when "c" is 1; "e" denotes an integer of 1 to 5, "f" denotes 0 or 1 and "g" is 1 or 2; and
the aminourazole compound (A3) comprises a 4-aminourazole compound shown by the following formula (3): 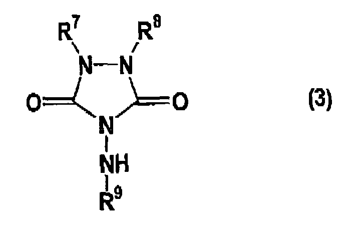 wherein R⁷, R⁸ and R⁹ are the same or different and each represents a hydrogen atom, an alkyl group, a cycloalkyl group, an aryl group or an aralkyl group;
or a salt thereof.

4. A deodorizing agent according to claim 3, wherein in the formula (1), R¹ is an alkyl group, a cycloalkyl group, a cycloalkylalkyl group, an aryl group, or an aralkyl group, R² is a hydrogen atom, R³ is a direct bond or a C₁₋₆alkylene group, "a" is an integer of 0 to 4, "b" is an integer of 1 to 3, and X¹ is a direct bond, a di- to pentavalent hydrocarbon group, or a di- or trivalent heterocyclic group.

5. A deodorizing agent according to claim 3, wherein the carboxylic acid hydrazide (A1) comprises a compound shown by the following formula (1a): 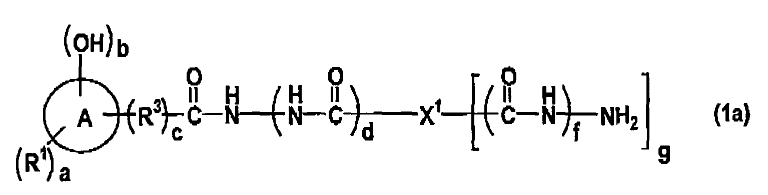 wherein R¹, R³, A, X¹, "a" to "d", "f" and "g" have the same meanings as defined above.

6. A deodorizing agent according to claim 1, wherein, R⁴, R⁵ and R⁶ in the formula (2) are alkylene groups and the polyhydrazide shown by the formula (2) has a number average molecular weight of 300 to 10,000.

7. A deodorizing agent according to claim 3, which comprises 4-aminourazole in which R⁷, R⁸ and R⁹ in the formula (3) are hydrogen atoms, a metal salt thereof, or a salt thereof with a nitrogen-containing compound.

8. A deodorizing agent according to claim 1, which further comprises at least one deodorizing auxiliary selected from the group consisting of a basic nitrogen-containing compound, a weak acid or a metal salt thereof, a metal compound, an alcohol, a polyphenol and an adsorbent inorganic compound.

9. A deodorizing agent according to claim 8, which comprises the deodorizing auxiliary in a proportion of 0.01 to 10,000 parts by weight relative to 100 parts by weight of the deodorizing agent.

10. A resin composition which comprises at least a deodorizing agent recited in claim 1 and a resin.

11. A resin composition according to claim 10, wherein the resin comprises at least one member selected from the group consisting of a polyacetal resin, an olefinic resin, a halogen-containing vinyl-series resin, a styrenic resin, an acrylic resin, a polyester-series resin, and a polyurethane-series resin.

12. A resin composition according to claim 10, which comprises 0.01 to 30 parts by weight of the deodorizing agent relative to 100 parts by weight of the resin.

13. A resin composition according to claim 10 or 11, which further comprises at least one member selected from the group consisting of an antioxidant, a heat stabilizer, a processing stabilizer, a weather (light)-resistant stabilizer, an impact resistance improver, a gloss-controlling agent, a slip-improving agent, a coloring agent, and a filler.

14. A polyacetal resin composition which comprises a polyacetal resin and at least a hydrazine compound recited in claim 1.

15. A resin composition according to claim 14, which further comprises at least one member selected from the group consisting of an antioxidant, a heat stabilizer, a processing stabilizer, a weather (light)-resistant stabilizer, an impact resistance improver, a gloss-controlling agent, a slip-improving agent, a coloring agent, and a filler.

16. A process for producing a polyacetal resin composition, which comprises melt-mixing a polyacetal resin and an hydrazine compound recited in claim 1 with an extruder, wherein at least the hydrazine compound is fed from a side feed port of the extruder and mixed with the polyacetal resin.

17. A process for producing a polyacetal resin composition, which comprises melt-mixing a polyacetal resin and a hydrazide compound recited in claim 1 with an extruder, wherein the average retention time in the extruder is 5 to 300 seconds.

18. A shaped article of a polyacetal resin, which is formed with a polyacetal resin composition recited in claim 14.

19. A shaped article according to claim 18, wherein (1) the emission of formaldehyde from the shaped article which is maintained in a closed space for 24 hours at a temperature of 80°C is not more than 1.0 µg per one cm² of the surface area of the article, and/or (2) the emission of formaldehyde from the shaped article which is maintained in a closed space for 3 hours at a temperature of 60°C under saturated humidity is not more than 1.2 µg per one cm² of the surface area of the article.

20. A shaped article according to claim 18, which is an automotive part, an electric or electronic device part, an architectural or pipeline part, a household utensil part, a medical device part, an office automation device part, a food grade part, or a photographic part.

## Patentansprüche

1. Aldehydgeruch-desodorierendes Mittel, das wenigstens eine Hydrazinverbindung umfasst, die aus der Gruppe ausgewählt ist, die aus einem Polycarbonsäurehydrazid mit einer Arylgruppe, einem Hydrazid einer Arylalkancarbonsäure (A1), einem nichtacrylischen Polyhydrazid (A2) und einer Aminourazolverbindung (A3) besteht;
wobei das Polycarbonsäurehydrazid eine Arylgruppe aufweist und das Hydrazid der Arylalkancarbonsäure (A1) wenigstens eine Hydroxygruppe oder eine von der Hydroxygruppe abgeleitete Gruppe an der bzw. den Arylgruppen aufweist;
wobei das Polycarbonsäurehydrazid eine Arylgruppe aufweist und das Hydrazid der Arylalkancarbonsäure (A1) wenigstens eine freie Hydrazinogruppe aufweist; und
wobei das nichtacrylische Polyhydrazid (A2) ein Polyhydrazid umfasst, das durch die folgende Formel (2) 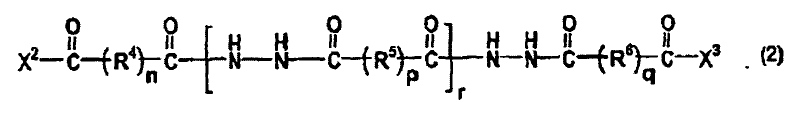 dargestellt wird, wobei R⁴ bis R⁶ gleich oder verschieden sind und jeweils eine zweiwertige Kohlenwasserstoffgruppe oder eine heterocyclische Gruppe darstellen, "n", "p" und "q" gleich oder verschieden sind und jeweils 0 oder 1 betragen, "r" eine ganze Zahl von nicht weniger als 0 bezeichnet;
X² und X³ gleich oder verschieden sind und jeweils ein Halogenatom, eine Aminogruppe, eine Hydrazinogruppe oder eine Gruppe -OX⁴ darstellen, wobei X⁴ ein Wasserstoffatom, eine Kohlenwasserstoffgruppe, ein Alkalimetallatom oder ein Erdalkalimetallatom darstellt, mit der Maßgabe, dass wenigstens eine der Gruppen X² und X³ eine Hydrazinogruppe ist.

2. Desodorierendes Mittel gemäß Anspruch 1, wobei das Polycarbonsäurehydrazid, das eine Arylgruppe aufweist, und das Hydrazid der Arylalkancarbonsäure (A1) wenigstens eine Gruppe aufweisen, die aus einer Hydroxygruppe, einer Alkoxygruppe und einer Acyloxygruppe an der bzw. den Arylgruppen ausgewählt ist.

3. Desodorierendes Mittel gemäß Anspruch 1, wobei das Polycarbonsäurehydrazid, das eine Arylgruppe aufweist, und das Hydrazid der Arylalkancarbonsäure (A1) ein Carbonsäurehydrazid aufweisen, das durch die folgende Formel (1) gezeigt ist: 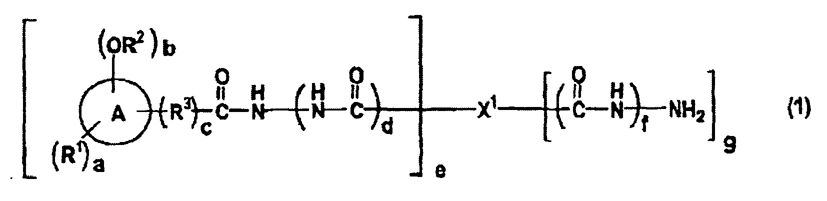 wobei R¹ eine Kohlenwasserstoffgruppe darstellt, R² ein Wasserstoffatom, eine Alkylgruppe oder eine Acylgruppe darstellt, R³ eine Alkylengruppe darstellt, Ring A einen aromatischen Ring darstellt, X¹ eine direkte Bindung oder eine mehrwertige organische Gruppe darstellt; "a" eine ganze Zahl von 0 bis 6 bezeichnet, "b" eine ganze Zahl von 1 bis 4 bezeichnet, "c" 0 oder 1 bezeichnet, "d" = 1 ist, wenn "c" = 0 ist, und "d" = 0 oder 1 ist, wenn "c" = 1 ist; "e" eine ganze Zahl von 1 bis 5 bezeichnet, "f" 0 oder 1 bezeichnet und "g" = 1 oder 2 ist; und
die Aminourazolverbindung (A3) eine 4-Aminourazolverbindung umfasst, die durch die folgende Formel (3) dargestellt wird: 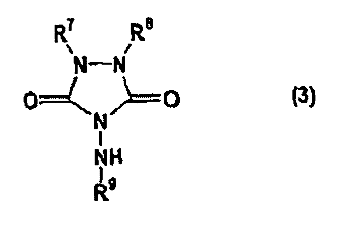 wobei R⁷, R⁸ und R⁹ gleich oder verschieden sind und jeweils ein Wasserstoffatom, eine Alkylgruppe, eine Cycloalkylgruppe, eine Arylgruppe oder eine Aralkylgruppe darstellen;
oder ein Salz davon.

4. Desodorierendes Mittel gemäß Anspruch 3, wobei in Formel (1) R¹ eine Alkylgruppe, eine Cycloalkylgruppe, eine Cycloalkylalkylgruppe, eine Arylgruppe oder eine Aralkylgruppe ist, R² ein Wasserstoffatom ist, R³ eine direkte Bindung oder eine C₁₋₆-Alkylengruppe ist, "a" eine ganze Zahl von 0 bis 4 ist, "b" eine ganze Zahl von 1 bis 3 ist und X¹ eine direkte Bindung, eine zwei- bis fünfwertige Kohlenwasserstoffgruppe oder eine zwei- oder dreiwertige heterocyclische Gruppe ist.

5. Desodorierendes Mittel gemäß Anspruch 3, wobei das Carbonsäurehydrazid (A1) eine Verbindung umfasst, die durch die folgende Formel (1a) dargestellt wird: 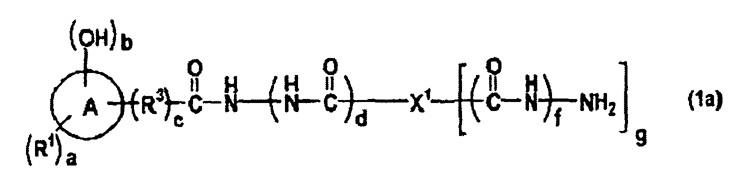 wobei R¹, R³, A, X¹, "a" bis "d", "f" und "g" dieselben Bedeutungen haben, wie sie oben definiert sind.

6. Desodorierendes Mittel gemäß Anspruch 1, wobei R⁴, R⁵ und R⁶ in Formel (2) Alkylengruppen sind und das durch Formel (2) dargestellte Polyhydrazid ein Zahlenmittel des Molekulargewichts von 300 bis 10 000 aufweist.

7. Desodorierendes Mittel gemäß Anspruch 3, das 4-Aminourazol umfasst, bei dem R⁷, R⁸ und R⁹ in Formel (3) Wasserstoffatome sind, ein Metallsalz davon oder ein Salz davon mit einer stickstoffhaltigen Verbindung.

8. Desodorierendes Mittel gemäß Anspruch 1, das weiterhin wenigstens ein desodorierendes Hilfsmittel umfasst, das aus der Gruppe ausgewählt ist, die aus einer basischen stickstoffhaltigen Verbindung, einer schwachen Säure oder einem Metallsalz davon, einer Metallverbindung, einem Alkohol, einem Polyphenol und einer adsorbierenden anorganischen Verbindung besteht.

9. Desodorierendes Mittel gemäß Anspruch 8, das das desodorierende Hilfsmittel in einem Anteil von 0,01 bis 10 000 Gewichtsteilen pro 100 Gewichtsteile des desodorierenden Mittels umfasst.

10. Harzzusammensetzung, die wenigstens ein desodorierendes Mittel gemäß Anspruch 1 und ein Harz umfasst.

11. Harzzusammensetzung gemäß Anspruch 10, wobei das Harz wenigstens einen Vertreter umfasst, der aus der Gruppe ausgewählt ist, die aus einem Polyacetalharz, einem olefinischen Harz, einem halogenhaltigen Harz der Vinylreihe, einem styrolischen Harz, einem acrylischen Harz, einem Harz der Polyesterreihe und einem Harz der Polyurethanreihe besteht.

12. Harzzusammensetzung gemäß Anspruch 10, die 0,01 bis 30 Gewichtsteile des desodorierenden Mittels pro 100 Gewichtsteile des Harzes umfasst.

13. Harzzusammensetzung gemäß Anspruch 10 oder 11, die weiterhin wenigstens einen Vertreter umfasst, der aus der Gruppe ausgewählt ist, die aus einem Antioxidationsmittel, einem Wärmestabilisator, einem Verarbeitungsstabilisator, einem witterungs(licht)beständigen Stabilisator, einem Schlagzähmacher, einem Glanzregulator, einem Gleitmittel, einem Farbstoff und einem Füllstoff besteht.

14. Polyacetalharzzusammensetzung, die ein Polyacetalharz und wenigstens eine Hydrazinverbindung gemäß Anspruch 1 umfasst.

15. Harzzusammensetzung gemäß Anspruch 14, die weiterhin wenigstens einen Vertreter umfasst, der aus der Gruppe ausgewählt ist, die aus einem Antioxidationsmittel, einem Wärmestabilisator, einem Verarbeitungsstabilisator, einem witterungs(licht)beständigen Stabilisator, einem Schlagzähmacher, einem Glanzregulator, einem Gleitmittel, einem Farbstoff und einem Füllstoff besteht.

16. Verfahren zur Herstellung einer Polyacetalharzzusammensetzung, das das Mischen eines Polyacetalharzes und einer Hydrazinverbindung gemäß Anspruch 1 in der Schmelze mit einem Extruder umfasst, wobei wenigstens die Hydrazinverbindung von einem Seiteneinfüllanschluss des Extruders her zugeführt und mit dem Polyacetalharz gemischt wird.

17. Verfahren zur Herstellung einer Polyacetalharzzusammensetzung, das das Mischen eines Polyacetalharzes und einer Hydrazidverbindung gemäß Anspruch 1 in der Schmelze mit einem Extruder umfasst, wobei die mittlere Retentionszeit in dem Extruder 5 bis 300 Sekunden beträgt.

18. Formkörper aus einem Polyacetalharz, der mit einer Polyacetalharzzusammensetzung gemäß Anspruch 14 gebildet wird.

19. Formkörper gemäß Anspruch 18, wobei (1) die Emission von Formaldehyd aus dem Formkörper, der 24 Stunden lang bei einer Temperatur von 80 °C in einem geschlossenen Raum gehalten wird, nicht mehr als 1,0 µg pro cm² der Oberfläche des Formkörpers beträgt und/oder (2) die Emission von Formaldehyd aus dem Formkörper, der 3 Stunden lang bei einer Temperatur von 60°C in einem geschlossenen Raum unter gesättigter Feuchtigkeit gehalten wird, nicht mehr als 1,2 µg pro cm² der Oberfläche des Formkörpers beträgt.

20. Formkörper gemäß Anspruch 18, bei dem es sich um ein Kraftfahrzeugteil, ein Teil einer elektrischen oder elektronischen Vorrichtung, ein Teil eines Bauwerks oder einer Rohrleitung, ein Teil eines Haushaltsutensils, ein Teil einer medizinischen Vorrichtung, ein Teil einer Büroautomatisierungsvorrichtung, ein lebensmitteltaugliches Teil oder ein photographisches Teil handelt.

## Revendications

1. Agent désodorisant à base d'aldéhyde qui comprend au moins un composé hydrazine choisi dans le groupe constitué par un hydrazide d'acide polycarboxylique ayant un groupe aryle, un hydrazide d'un acide arylalcanecarboxylique (A1), un polyhydrazide non acrylique (A2), et un composé aminourazole (A3),
dans lequel l'hydrazide d'acide polycarboxylique ayant un groupe aryle et l'hydrazide de l'acide arylalcanecarboxylique (A1) ont au moins un groupe hydroxyle ou un groupe dérivé du groupe hydroxy sur le ou les groupe(s) aryle(s),
l'hydrazide d'acide polycarboxylique ayant un groupe aryle et l'hydrazide de l'acide arylalcanecarboxylique (A1) ont au moins un groupe hydrazino libre, et
le polyhydrazide non acrylique (A2) comprend un polyhydrazide illustré par la formule (2) suivante : 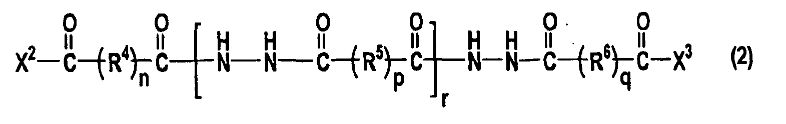 dans laquelle R⁴ à R⁶ sont identiques ou différents et représentent chacun un groupe hydrocarboné divalent ou un groupe hétérocyclique, "n", "p" et "q" sont identiques ou différents et représentent chacun 0 ou 1, "r" représente un nombre entier non inférieur à 0 ;
X² et X³ sont identiques ou différents et représentent chacun un atome d'halogène, un groupe amino, un groupe hydrazino, ou un groupe -OX⁴ où X⁴ représente un atome d'hydrogène, un groupe hydrocarboné, un atome de métal alcalin, ou un atome de métal alcalino-terreux, et à condition qu'au moins un des X² et X³ soit un groupe hydrazino.

2. Agent désodorisant selon la revendication 1, dans lequel l'hydrazide d'acide polycarboxylique ayant un groupe aryle et l'hydrazide de l'acide arylalcanecarboxylique (A1) ont au moins un groupe choisi parmi un groupe hydroxyle, un groupe alcoxy et un groupe acyloxy sur le ou les groupe(s) aryle(s).

3. Agent désodorisant selon la revendication 1, dans lequel l'hydrazide d'acide polycarboxylique ayant un groupe aryle et l'hydrazide de l'acide arylalcanecarboxylique (A1) comprend un hydrazide d'acide carboxylique illustré par la formule (1) suivante : 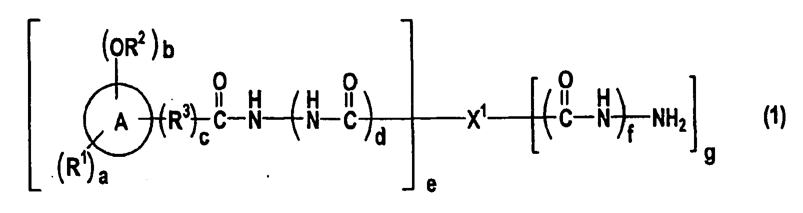 dans laquelle R¹ représente un groupe hydrocarboné, R² représente un atome d'hydrogène, un groupe alkyle ou un groupe acyle, R³ représente un groupe alkylène, un cycle A représente un cycle aromatique, X¹ représente une liaison directe ou un groupe organique polyvalent ; "a" représente un nombre entier de 0 à 6 ; "b" représente un nombre entier de 1 à 4 ; "c" représente 0 ou 1, "d" représente 1 lorsque "c" vaut 0, "d" représente 0 ou 1 lorsque "c" vaut 1 ; "e" représente un nombre entier de 1 à 5 ; "f" représente 0 ou 1 et "g" représente 1 ou 2 ; et
le composé aminourazole (A3) comprend un composé 4-aminourazole illustré par la formule (3) suivante : 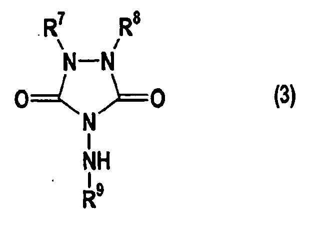 dans laquelle R⁷, R⁸ et R⁹ sont identiques ou différents et représentent chacun un atome d'hydrogène, un groupe alkyle, un groupe cycloalkyle, un groupe aryle ou un groupe aralkyle ;
ou un sel de celui-ci.

4. Agent désodorisant selon la revendication 3, dans lequel dans la formule (1), R¹ est un groupe alkyle, un groupe cycloalkyle, un groupe cycloalkylalkyle, un groupe aryle, ou un groupe aralkyle, R² est un atome d'hydrogène, R³ est une liaison directe ou un groupe alkylène en C₁-C₆, "a" représente un nombre entier de 0 à 4 ; "b" représente un nombre entier de 1 à 3, et X¹ est une liaison directe, un groupe hydrocarboné di- à pentavalent, ou un groupe hétérocyclique di- ou trivalent.

5. Agent désodorisant selon la revendication 3, dans lequel l'hydrazide d'acide carboxylique (A1) comprend un composé illustré par la formule (la) suivante : 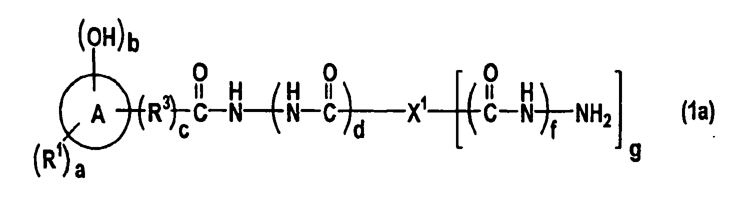 dans laquelle R¹, R³, A, X¹, "a" à "d", "f" et "g" ont les mêmes significations que celles définies ci-dessus.

6. Agent désodorisant selon la revendication 1, dans lequel R⁴, R⁵ et R⁶ dans la formule (2) sont des groupes alkylènes et le polyhydrazide illustré par la formule (2) a un poids moléculaire moyen en nombre de 300 à 10000.

7. Agent désodorisant selon la revendication 3, qui comprend le 4-aminourazole
dans lequel R⁷, R⁸ et R⁹ dans la formule (3) sont des atomes d'hydrogène, un sel métallique de celui-ci, ou un sel de celui-ci avec un composé contenant de l'azote.

8. Agent désodorisant selon la revendication 1, qui comprend en outre au moins un auxiliaire désodorisant choisi dans le groupe constitué par un composé basique contenant de l'azote, un acide faible ou un métal de celui-ci, un composé métallique, un alcool, un polyphénol et un composé organique adsorbant.

9. Agent désodorisant selon la revendication 8, qui comprend l'auxiliaire désodorisant en une proportion de 0,01 à 10000 parties en poids par rapport à 100 parties en poids de l'agent désodorisant.

10. Composition de résine qui comprend au moins un agent désodorisant selon la revendication 1 et une résine.

11. Composition de résine selon la revendication 10, dans laquelle la résine comprend au moins un élément choisi dans le groupe constitué par une résine polyacétal, une résine oléfinique, un résine vinylique contenant de l'atome d'halogène, une résine styrénique, une résine acrylique, une résine polyester, et une résine polyuréthane.

12. Composition de résine selon la revendication 10, qui comprend de 0,01 à 30 parties en poids de l'agent désodorisant par rapport à 100 parties en poids de la résine.

13. Composition de résine selon la revendication 10 ou 11, qui comprend en outre au moins un élément choisi dans le groupe constitué par un antioxydant, un stabilisateur thermique, un stabilisateur de traitement, un stabilisateur de résistance aux intempéries (à la lumière), un améliorateur de résistance à l'impact, un agent de contrôle de la brillance, un agent améliorant le glissage, un agent colorant et une charge.

14. Composition de résine polyacétal qui comprend une résine polyacétal et au moins un composé hydrazine selon la revendication 1.

15. Composition de résine selon la revendication 14, qui comprend en outre au moins un élément choisi dans le groupe constitué par un antioxydant, un stabilisateur thermique, un stabilisateur de traitement, un stabilisateur de résistance aux intempéries (à la lumière), un améliorateur de résistance à l'impact, un agent de contrôle de la brillance, un agent améliorant le glissage, un agent colorant et une charge.

16. Procédé de production d'une composition de résine polyacétal, qui comprend le mélange à l'état fondu d'une résine polyacétal et d'un composé hydrazine selon la revendication 1 avec une extrudeuse, dans lequel au moins le composé hydrazine est alimenté par un port d'alimentation latéral de l'extrudeuse et mélangé avec la résine polyacétal.

17. Procédé de production d'une composition de résine polyacétal, qui comprend le mélange à l'état fondu d'une résine polyacétal et d'un composé hydrazine selon la revendication 1 avec une extrudeuse, dans lequel le temps de rétention moyen dans l'extrudeuse est de 5 à 300 secondes.

18. Article façonné en résine polyacétal, qui est formé avec une composition de résine polyacétal selon la revendication 14.

19. Article façonné selon la revendication 18, dans lequel (1) l'émission de formaldéhyde de l'article façonné qui est maintenu dans un espace fermé pendant 24 heures à une température de 80 °C n'est pas supérieure à 1,0 µg par cm² de la surface de l'article, et/ou (2) l'émission de formaldéhyde à partir de l'article façonné qui est maintenu dans un espace fermé pendant 3 heures à une température de 60 °C dans une humidité saturée n'est pas supérieure à 1,2 µg par cm² de la surface de l'article.

20. Article façonné selon la revendication 18, qui est une partie d'une automobile, un partie d'un appareil électrique ou électronique, un partie architecturale ou de pipeline, une partie d'un ustensile ménager, une partie d'un appareil médical, une partie d'un appareil bureautique, une partie de classe alimentaire, ou une partie photographique.
